(19) Europäisches Patentamt
European Patent Office
Office européen des brevets

(11) EP 4 617 380 A1

(12) EUROPEAN PATENT APPLICATION

(43) Date of publication:
**17.09.2025 Bulletin 2025/38**

(21) Application number: **25163778.1**

(22) Date of filing: **14.03.2025**

(51) International Patent Classification (IPC):
***C12Q 1/6816*** *(2018.01)*

(52) Cooperative Patent Classification (CPC):
(C-Sets available)
**C12Q 1/6816** (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **15.03.2024 KR 20240036772**

(71) Applicants:
- **Sogang University Research & Business Development Foundation Seoul 04107 (KR)**
- **Research & Business Foundation SungKyunKwan University Gyeonggi-do 16419 (KR)**

(72) Inventors:
- **JO, Kyubong Seoul (KR)**
- **LEE, Jung Heon Suwon-si (KR)**
- **NOH, Chanyoung Seoul (KR)**
- **RAMASAMI SUNDHARBAABU, Priyannth Suwon-si (KR)**
- **KANG, Yoonjeong Seoul (KR)**
- **CHANG, Junhyuck Suwon-si (KR)**

(74) Representative: **Appleyard Lees IP LLP 15 Clare Road Halifax HX1 2HY (GB)**

Remarks:
The complete document including Reference Table(s) and the Sequence Listing(s) can be downloaded from the EPO website

# (54) METHOD FOR SCANNING ELECTRON MICROSCOPY (SEM)-BASED OPTICAL DNA MAPPING

(57) Provided is a method of scanning electron microscopy (SEM)-based optical DNA mapping. The method of optical DNA mapping according to an aspect, by utilizing SEM as a basic platform, provides enhanced accessibility and user-friendliness, superior compatibility with chemically functionalized surfaces and microfluidic devices, and the ability to obtain high-resolution DNA images without metal usage, thereby enabling optical DNA mapping with improved resolution.

FIG. 1

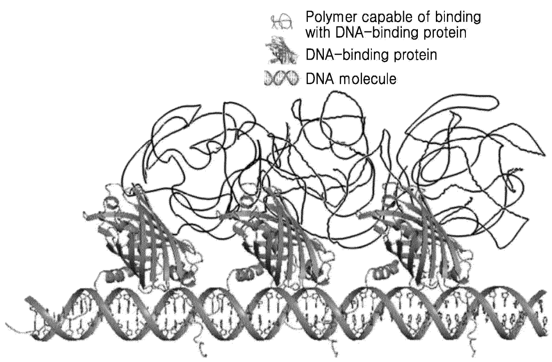




Processed by Luminess, 75001 PARIS (FR)

(Cont. next page)

(52) Cooperative Patent Classification (CPC): (Cont.)

C-Sets
**C12Q 1/6816, C12Q 2565/601, C12Q 2521/514,**
**C12Q 2521/319, C12Q 2563/107**

## Description

### CROSS-REFERENCE TO RELATED APPLICATION

This application is based on and claims priority under 35 U.S.C. §119 to Korean Patent Application No. 10-2024-0036772, filed on March 15, 2024, in the Korean Intellectual Property Office, the disclosure of which is incorporated by reference herein in its entirety.

### BACKGROUND

#### 1. Field

The present disclosure relates to a method of scanning electron microscopy (SEM)-based optical DNA mapping.
The research described herein was conducted with support from the Samsung Future Technology Fostering Project (Project No. SRFC-MA2101-07).

#### 2. Description of the Related Art

Optical DNA mapping is a technique for constructing ordered, genome-wide maps from single, stained molecules of DNA. Single-molecule DNA sequencing technology is rapidly advancing, but generally relies on reading the genome in fragments smaller than 1000 bp. The main purpose of optical DNA mapping is to complement DNA sequence analysis by providing a large-scale scaffold onto which these short sequence reads can be assembled. Specifically, optical DNA mapping, when combined with base-by-base sequencing methods, can guide the assembly of complex genomic regions. This makes it a useful tool for reaching DNA length scales inaccessible to conventional sequencing. Such optical DNA mapping requires imaging techniques capable of visualizing DNA molecules with high effectiveness.
Methods for DNA analysis and observation in the related art include microscopic techniques such as transmission electron microscopy (TEM), fluorescence microscopy (FM), and scanning electron microscopy (SEM). While transmission electron microscopy (TEM) enables detailed observation of the fine structure of DNA, it requires DNA imaging performed by using heavy metal salts, such as uranyl acetate, in combination with a shadow casting method to overcome low electron scattering. To this end, much time and complicated processes are required to observe DNA molecules through TEM. Fluorescence microscopy (FM) is widely used due to its simplicity, accessibility, and live imaging capabilities, but its inherent limitation of low resolution makes accurate observation of DNA molecules at the nanometer scale challenging. One of the key requirements for DNA imaging is compatibility with microfluidic devices and chemically functionalized surfaces. While DNA images obtained by FM typically show well-aligned and parallel extension, TEM faces difficulties in achieving such alignment due to alignment issues with carbon film-coated metal grids, resulting in generally randomly arranged DNA molecules. SEM can achieve both nanometer-scale resolution and millimeter-scale observation range. Additionally, it offers greater flexibility in accommodating functionalized surfaces and microfluidic devices for DNA molecule alignment. Therefore, SEM is considered a method capable of addressing the drawbacks of both transmission electron microscopy and fluorescence microscopy. However, SEM has a limitation in that its resolution far exceeds the 2 nm thickness of DNA. Various methods have been developed to enhance the visibility of DNA molecules, including nanoparticle attachment and nanowire growth. However, these approaches primarily serve to visualize artificially constructed DNA nanostructures and encounter substantial challenges when applied to genomic DNA.
Consequently, there is a need for a new optical DNA mapping technique that can achieve high-resolution images comparable to TEM while retaining the many advantages of FM, such as simplicity, accessibility, and the capability to obtain well-aligned DNA images.

### SUMMARY

An aspect is to provide a method of scanning electron microscopy (SEM)-based optical DNA mapping, including: (a) contacting a double-stranded DNA with a nickase to form a nick at a target sequence motif; (b) generating labeled double-stranded DNA by incorporating labeled nucleotides into a nick site of the double-stranded DNA; (c) staining a backbone of the labeled double-stranded DNA; (d) stretching and immobilizing the stained double-stranded DNA on a substrate having a functionalized surface; and (e) determining the distance between each label by imaging the immobilized double-stranded DNA using a scanning electron microscopy (SEM).
Additional aspects will be set forth in part in the description which follows and, in part, will be apparent from the description, or may be learned by practice of the presented embodiments of the disclosure.
An aspect provides a method of scanning electron microscopy (SEM)-based optical DNA mapping, including: (a)

contacting a double-stranded DNA with a nickase to form a nick at a target sequence motif; (b) generating labeled double-stranded DNA by incorporating labeled nucleotides into a nick site of the double-stranded DNA; (c) staining a backbone of the labeled double-stranded DNA; (d) stretching and immobilizing the stained double-stranded DNA on a substrate having a functionalized surface; and (e) determining the distance between each label by imaging the immobilized double-stranded DNA using a scanning electron microscopy (SEM).

The term "optical DNA mapping" refers to a technology that uses an imaging technique to provide positional information covering the entire genome from a single DNA molecule. Such positional information may be combined with sequence analysis data to serve as a guideline for genome sequence assembly.

The term "scanning electron microscopy (SEM)" refers to an apparatus that performs observation of microstructures by scanning a specimen with an electron beam, detecting signals emanating from the specimen surface with a detector, and displaying their intensity as a three-dimensional image on a monitor to obtain an enlarged image of the specimen. The scanning electron microscope may include a thermionic-emission SEM or a field-emission SEM.

A step-by-step explanation of the method of SEM-based optical DNA mapping is provided below.

First, the method may include (a) contacting a double-stranded DNA with a nickase to form a nick at a target sequence motif; and (b) generating labeled double-stranded DNA by incorporating labeled nucleotides into a nick site of the double-stranded DNA.

Steps (a) and (b) are steps for sequence-specific labeling of DNA molecules via nick translation.

The term "nickase" refers to an endonuclease that cleaves only one strand of double-stranded DNA, that is, introduces a nick into double-stranded DNA.

The nickase may be a nicking endonuclease, and the term "nicking endonuclease" refers to an endonuclease that recognizes a target sequence motif to form a sequence-specific nick. The nicking endonuclease may be naturally occurring, or may be engineered from a restriction endonuclease. In an embodiment, the nicking endonuclease may be Nb.BbvCl, Nb.BssSI, Nb.Bsml, Nb.BsrDl, Nb.Btsl, Nt.Alwl, Nt.BbvCl, Nt.BsmAl, Nt.BspQI, Nt.BstNBI, or Nt.CviPll, but is not limited thereto. Any endonuclease capable of recognizing a target sequence motif and forming a nick in a double-stranded DNA molecule may be used in accordance with the method of the present disclosure. More than 200 nicking endonucleases have been studied, and some of these are commercially available from sources such as New England Biolabs. The target sequence motif may be a short nucleotide sequence of 8 or fewer bases. For example, Nb.BbvCl recognizes and cleaves at the 5'-CC^TCAGC-3' site, and Nb.BssSI recognizes and cleaves at the 5'-C^TCGTG-3' site.

Alternatively, the nickase may be an RNA-guided endonuclease, and the term "RNA-guided endonuclease" refers to an endonuclease that recognizes a target sequence motif through guide RNA (gRNA) and forms a sequence-specific nick by forming a complex with the guide RNA. The RNA-guided endonuclease may be, for example, nCAS9 (cas9 nickase), and when the nickase is an RNA-guided endonuclease, the nickase may be used with a guide RNA sequence that includes a portion of a sequence complementary to the target double-stranded DNA.

In an embodiment, the nickase may be Nb.BbvCl, Nb.Bsml, Nb.BsrDI, Nb.Btsl, Nt.Alwl, Nt.BbvCl, Nt.BsmAl, Nt.BspQI, Nt.BstNBI, Nt.CviPll, nCAS9, or a combination thereof.

The term "nick" refers to a broken phosphodiester bond site in double-stranded DNA, and introducing a nick can expose free-3' and free-5' strands at that site. That is, the nick may include a 3' hydroxyl and an adjacent 5' phosphate.

The term "nick translation" refers to a process in which a polymerase extends a DNA strand from the 3' hydroxyl at the nick site while simultaneously degrading the adjacent strand.

In an embodiment, the labeled nucleotide may be a nucleotide to which a detectable label is conjugated either directly or indirectly. The detectable label may be conjugated to the nucleotide via covalent bonding or via binding pairs of a first binding moiety and a second binding moiety.

The term "detectable label" refers to a label detectable by optical methods, that is, an optical label. The detectable label may be a label detectable under SEM, and may be a label that can be visualized brightly in contrast to DNA backbones that appear as dark lines under SEM through staining steps.

In an embodiment, the detectable label may be a nanoparticle or a fluorescent dye. The nanoparticle may be any one selected from the group consisting of a metal nanoparticle, an oxide nanoparticle, a sulfide nanoparticle, a nanocluster, a quantum dot, a graphene quantum dot, a perovskite, a carbon dot, a polymer particle, hydroxyapatite, and a magnetic nanoparticle. The metal nanoparticle may be, for example, a gold nanoparticle. The fluorescent dye may be, for example, a cyanine dye (e.g., Cy3, Cy5, and Cy7 dyes); xanthene dyes (e.g., fluorescein isothiocyanate (FITC), 6-carboxyfluorescein, 6-carboxy-2',4',7,4,7-hexachlorofluorescein (HEX), 6-carboxy-X-rhodamine (ROX or R), 5-carboxyrhodamine 6G (R6G5 or G5), 6-carboxyrhodamine-6G (R6G6 or G6), and rhodamine 110 and other fluorescein and rhodamine dyes); and benzimide dyes (e.g., Hoechst 33258); phenanthridine dyes (e.g., Texas Red). The diameter of the nanoparticle may be 5 nm to 20 nm. When the diameter of the nanoparticle exceeds the aforementioned range, introduction at the nick site may be difficult, and when the diameter is below the aforementioned range, observation under SEM may be difficult.

In an embodiment, step (b) may include: contacting the nicked double-stranded DNA with a DNA polymerase in the presence of at least one first binding-moiety-conjugated nucleotide to generate a modified double-stranded DNA

having a first binding moiety; and contacting the modified double-stranded DNA with a detectable label conjugated to a second binding moiety to generate labeled double-stranded DNA, wherein the second binding moiety forms a binding pair with the first binding moiety.

In an embodiment, the first binding moiety or the second binding moiety may be any one selected from the group consisting of biotin, streptavidin, digoxin, neutravidin, and avidin. For instance, the first binding moiety and the second binding moiety may form a binding pair of biotin/streptavidin, biotin/neutravidin, or biotin/avidin.

In an embodiment, the first binding-moiety-conjugated nucleotide may be biotin-dUTP, and the detectable label conjugated to the second binding moiety may be a detectable label coated with streptavidin.

In an embodiment, the DNA polymerase may have 5'→3' exonuclease activity. This DNA polymerase may remove original nucleotides in the 5'→3' direction while simultaneously replacing them with labeled nucleotides via its 5'→ 3' polymerase activity. The DNA polymerase may be DNA polymerase I.

The step of generating the modified double-stranded DNA may involve contacting the double-stranded DNA with one or more first-binding-moiety-conjugated nucleotides, one or more nucleotides (dNTPs-dTTP, dATP, dCTP, and dGTP), a DNA polymerase, or a ligase.

In addition, the method may include (c) staining a backbone of the labeled double-stranded DNA.

This step is a step for electro-staining DNA for visualization under scanning electron microscope (SEM).

FIG. 1 is a schematic diagram illustrating DNA electro-staining using DNA-binding proteins and polymers according to an embodiment.

According to an aspect, the staining reagent used in the method may be prepared by binding a DNA-binding protein with a synthetic polymer, and referring to FIG. 1, DNA may be stained by forming a complex with the DNA-binding protein and a polymer capable of binding to the DNA.

In an embodiment, step (c) may include contacting the labeled double-stranded DNA with a DNA-binding protein.

The DNA binding protein may bind a DNA molecule through one or more of the following interactions: electrostatic interaction, intercalation, or groove binding. By way of example, a peptide in an embodiment may include positively charged amino acid residues that electrostatically interact with the negatively charged DNA molecule. The peptide that binds with DNA molecules through electrostatic interaction may include an amino acid sequence containing at least one selected from arginine, histidine, and lysine. Groove binding refers to the interaction of exposed bases on the DNA molecule with the peptide that includes the amino acid sequence, such that the peptide can bind DNA through groove binding. Intercalation is a phenomenon in which molecules, atoms, or ions are inserted between the layers of a layered material; therefore, a peptide containing the above amino acid sequence can be inserted into the DNA molecule. In such a case, a peptide that binds the DNA molecule through intercalation may include an amino acid sequence containing at least one aromatic amino acid ring selected from phenylalanine, tyrosine, and tryptophan.

The peptide may carry out groove binding or intercalation with a DNA molecule, in addition to electrostatic interactions, or may perform both intercalation and groove binding along with electrostatic interactions.

The peptide may have one or more functional groups at-at least one of its N-terminus or C-terminus. Such functional groups may include one or more selected from thiol groups, amine groups, carboxyl groups, cysteine groups, azide groups, alkyne groups, glutaraldehyde groups, and maleimide groups, but are not limited thereto and may encompass all peptides usable in DNA-binding proteins. For example, the peptide may bind to a DNA molecule and thereby provide the aforementioned functional groups to the DNA molecule.

In an embodiment, the DNA-binding protein may include a truncated transcription activator-like effector (tTALE), a histone-like nucleoid-structural protein (H-NS), Cro, or a high mobility group (HMG) protein, among others.

In an embodiment, the DNA-binding protein may further include a fluorescent protein. A DNA-binding protein such as H-NS, or a fluorescent protein, may serve as an excellent docking site for the polymer described below. In addition, oligomerization of the fluorescent protein may enhance binding strength with the polymer described below, and may exist as an assembly around the DNA molecule together with the polymer described below.

In an embodiment, the fluorescent protein may include mScarlet, EmGFP, mNeonGreen, RRvT, tdTomato, mClover3, YPet, mOrange2, Emerald, mTurquoise2, mStrawberry, and mCherry, among others.

In an embodiment, the DNA-binding protein may be joined to a fluorescent protein via a linker, or the DNA-binding protein (DBP) and fluorescent protein (FP) may be directly joined without a linker. Examples of such DNA-binding protein-fluorescent proteins may include truncated TALE (tTALE)-emGFP, H-NS-mScarlet, Cro-mNeonGreen, and mNeon-Green-HMG, among others. Such DNA-binding protein-fluorescent proteins may influence both the brightness required for visualizing DNA molecules by electron microscopy and the extension of DNA molecules.

In an embodiment, step (c) may further include contacting the double-stranded DNA with a polymer capable of binding the DNA-binding protein, an anhydrate thereof, or a salt thereof.

The polymer capable of binding the DNA-binding protein may include an anhydrate thereof or a salt thereof in addition to the polymer itself. The salt may be an inorganic salt or an organic salt. For example, the salt may be a cationic inorganic salt.

The polymer may be capable of binding through intermolecular electrostatic attraction with the DNA binding

protein.

According to an embodiment, the polymer may include one or more atoms of oxygen and nitrogen.

According to an embodiment, the oxygen atom or nitrogen atom of the polymer may bind through hydrogen bonding with hydrogen atoms of O-H bonds or N-H bonds included in the DNA-binding protein. This hydrogen bonding corresponds to intermolecular bonding and is distinct from chemical bonding.

According to an embodiment, the polymer, the anhydrate thereof, or the salt thereof, may be in the form of aggregates.

In an embodiment, the polymer may include one or more structural units represented by Formulas 1 to 9 below:

[Formula 1]

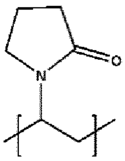

,

[Formula 2]

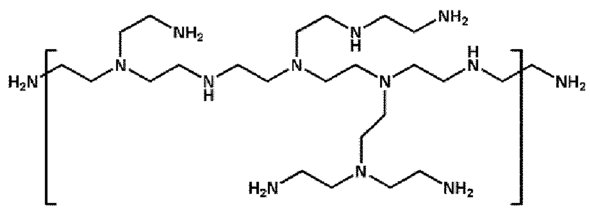

,

[Formula 3]

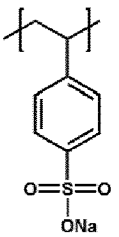

,

[Formula 4]

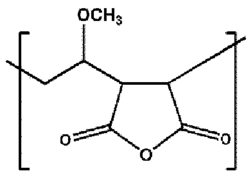

,

[Formula 5]

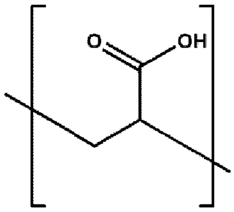

,

[Formula 6]

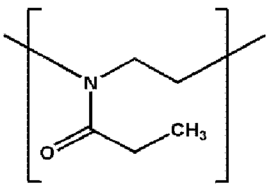

,

[Formula 7]

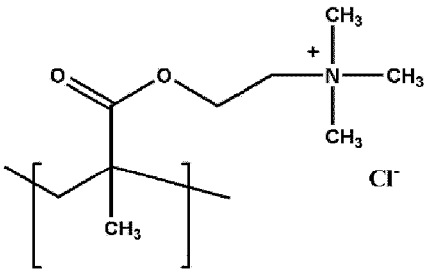

,

[Formula 8]

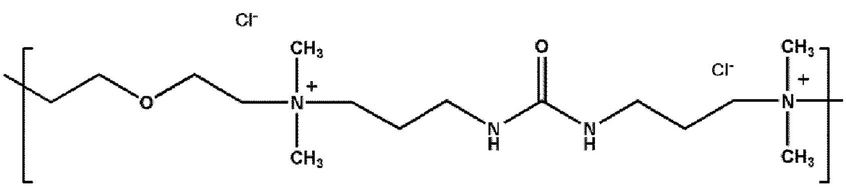

,

[Formula 9]

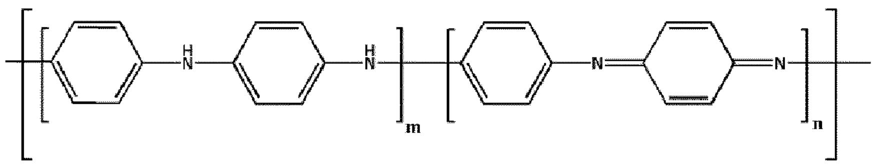

wherein in Formulas 1 to 9,
m + n may be equal to 1.

In an embodiment, the weight-average molecular weight (Mw) of the polymer, the anhydrate thereof, or the salt thereof, may be 10 kDa to 100 kDa, for example, may be 20 kDa to 100 kDa, 30 kDa to 100 kDa, 40 kDa to 100 kDa, 10 kDa to 90 kDa, 20 kDa to 90 kDa, 30 kDa to 90 kDa, 40 kDa to 90 kDa, 10 kDa to 60 kDa, 20 kDa to 60 kDa, 30 kDa to 60 kDa, 40 kDa to 60 kDa, or 30 kDa to 50 kDa.

According to an embodiment, when used in conjunction with the aforementioned DNA-binding protein, the polymer, the anhydrate thereof, or the salt thereof, can stain DNA molecules in black at an average thickness of about 15.0 $\pm$ 4.0 nm, thereby facilitating visualization of DNA molecules by SEM. As confirmed in an embodiment, this method enables high-resolution imaging not only of linear, circular, single-stranded, or double-stranded DNA configurations, but also of a broad range of DNA structures-from genomic and mitochondrial DNA to chromatin and chromosomes.

Because the method according to an aspect can enhance the visibility of 2 nm-thick DNA molecules for SEM imaging without using heavy metals such as uranyl acetate, which is conventionally used as an electron-microscopy staining reagent, step (c) may be a metal-free staining step. Thus, unlike transmission electron microscopy (TEM), this approach does not require extensive time or complicated procedures to observe DNA molecules. Moreover, in contrast to most electrostaining techniques, the method according to an aspect conducts staining without the use of metal, causing DNA to appear as a continuous dark line under SEM. As a result, it is possible to obtain high-resolution SEM images of various DNA forms-including linear, circular, double-stranded, single-stranded, and even RNA-and to visualize human cellular genomic and mitochondrial DNA molecules, as well as transcriptional chromatin and chromosomes.

In addition, the method may further include (d) stretching and immobilizing the stained double-stranded DNA on a substrate having a functionalized surface.

In an embodiment, the substrate having a functionalized surface may be a silicon substrate positively charged via surface modification. The silicone substrate may be a silicon substrate having an $SiO_2$ layer formed thereon. The surface modification may be performed with an ammonium salt. The silicon substrate may be advantageously used to accommodate a PDMS microfluidic device with ease.

The positively charged, surface-modified silicon substrate serves to immobilize DNA molecules. In contrast, a glass substrate surface-commonly used for fluorescence microscopy (FM)-induces electron charging effects and is thus unsuitable for observing DNA molecules by SEM. Furthermore, a substrate coated with a conductive metal such as gold (Au) or platinum (Pt) blurs the 2 nm-thick DNA image under SEM, rendering such a substrate unsuitable for DNA observation. The silicon substrate used in the present disclosure, due to its semiconductor characteristics, enables effective charge transfer.

The positively charged, surface-modified silicon substrate may be prepared by bringing an ammonium salt precursor into contact with a silicon substrate having an $SiO_2$ layer formed thereon. The silicon substrate having an $SiO_2$ layer formed thereon and modified with positive charges from an ammonium salt serves to immobilize the DNA molecule backbone. The thickness of the $SiO_2$ layer may be in the range of several nanometers or tens of nanometers. The thickness of the $SiO_2$ layer may be appropriately adjusted within a range that maintains positive charges after surface modification with positive charges.

The ammonium salt precursor may include one or more selected from ammonium hydroxide, ammonium chloride compounds, ammonium sulfate compounds, ammonium carbonate compounds, ammonium bicarbonate compounds, and ammonium acetate compounds. For example, the ammonium salt precursor may be an ammonium chloride compound. For example, the ammonium salt precursor may be N-trimethoxysilylpropyl-N,N,N-trimethylammonium chloride.

In an embodiment, step (d) may include passing the stained double-stranded DNA through a microchannel. Various nucleic acid stretching methods known in the related art may be used to linearly stretch DNA. According to an embodiment, a microfluidic device can provide uniform and efficient DNA stretching through microchannels, resulting in well-aligned parallel images when observed through scanning electron microscope (SEM). DNA stretching through the microchannels may be used to maximize information obtainable from optical DNA mapping. The microchannel may be a polydimethylsiloxane (PDMS) microchannel, which functions to guide the DNA solution-having undergone labeling and staining-onto the silicon substrate surface. In an embodiment, step (d) may further include drying before removing the microchannel from the substrate. For example, the DNA loaded on the silicon substrate surface may be dried at room temperature for about 30 minutes to 1 hour, thereby sufficiently immobilizing the DNA, after which imaging can be performed via SEM.

Because the method of mapping according to an aspect employs SEM as the basic platform, this method may be seamlessly integrated with chemically functionalized surfaces and microfluidic devices.

Additionally, the method may include (e) determining the distance between each label by imaging the immobilized double-stranded DNA using a SEM.

This step involves detecting labeling patterns on the stretched double-stranded DNA through SEM imaging, thereby generating positional information.

## BRIEF DESCRIPTION OF THE DRAWINGS

The above and other aspects, features, and advantages of certain embodiments of the disclosure will be more apparent from the following description taken in conjunction with the accompanying drawings, in which:

FIG. 1 is a schematic diagram illustrating DNA electrostaining using a DNA-binding protein and a polymer, according to an embodiment;

FIG. 2 is a schematic diagram of a PDMS microfluidic device according to an embodiment;

FIG. 3 is an SEM image (untreated control) of bacteriophage λ DNA molecules obtained by stretching/immobilizing and imaging in the same manner as in Example 1-1, but without any staining reagent;

FIG. 4 shows an SEM image (Comparative Example 1-1) of bacteriophage λ DNA molecules stained using mNG-HMG alone and then stretched/immobilized and imaged, along with a corresponding FM image;

FIG. 5 is an SEM image (Comparative Example 1-2) of bacteriophage λ DNA molecules stained using PVP alone and then stretched/immobilized and imaged;

FIG. 6 is an SEM image (Example 1-2) of bacteriophage λ DNA molecules stained using H-NS and PVP in combination, followed by stretching/immobilizing and imaging;

FIG. 7 shows an SEM image (Example 1-3) of bacteriophage λ DNA molecules stained using H-NS-mScarlet and PVP in combination, followed by stretching/immobilizing and imaging, and also shows a corresponding FM image;

FIG. 8 shows an SEM image (Example 1-4) of bacteriophage λ DNA molecules stained using tTALE-EmGFP and PVP in combination, followed by stretching/immobilizing and imaging, along with a corresponding FM image;

FIG. 9 shows an SEM image (Example 1-5) of bacteriophage λ DNA molecules stained using Cro-mNeonGreen and PVP in combination, followed by stretching/immobilizing and imaging, along with a corresponding FM image;

FIG. 10 shows an SEM image (Example 1-6) of bacteriophage λ DNA molecules stained using mNG-HMG and poly(2-ethyl-2-oxazoline) in combination, followed by stretching/immobilizing and imaging, and also shows an SEM image of bacteriophage λ DNA molecules stained using the same combination, and immobilized on a positively charged, surface-modified silicon wafer without using a microfluidic device, and imaged;

FIG. 11 shows an SEM image (Example 1-7) of bacteriophage λ DNA molecules stained using mNG-HMG and polyaniline in combination, followed by stretching/immobilizing and imaging;

FIG. 12 shows an SEM image (Comparative Example 1-3) of bacteriophage λ DNA molecules stained using polyaniline alone, and then immobilized on a positively charged, surface-modified silicon wafer without using a microfluidic device;

FIG. 13 illustrates the SEM visualization of PVP droplets on various functionalized silicon wafer surfaces, namely, a positively charged, surface-modified silicon wafer, a silicon wafer having a $SiO_2$ layer, a plain silicon wafer, and a platinum-coated silicon wafer, wherein the dashed line in the platinum-coated wafer image indicates the boundary of the PVP droplet;

FIGS. 14A and 14B respectively show the SEM imaging result and the corresponding FM imaging result for bacteriophage λ DNA molecules stained using mNG-HMG and PVP in combination, and then applied and immobilized onto the surface of a positively charged, surface-modified silicon wafer without using a microfluidic device, and

imaged;

FIG. 15 shows the SEM imaging result (Example 1-1) for bacteriophage λ DNA molecules stained using mNG-HMG and PVP in combination, followed by stretching/immobilizing and imaging;

FIG. 16A shows the SEM imaging result (Example 1-1) for bacteriophage λ DNA (48.5 kb) molecules stained using mNG-HMG and PVP in combination, followed by stretching/immobilizing and imaging, along with the corresponding FM imaging result, and FIG. 16B shows an inverted, enlarged version of the SEM image from FIG. 16A for the bacteriophage λ DNA (48.5 kb), including the intensity profile measured along the yellow line of the inverted SEM DNA image and the corresponding full width at half maximum (FWHM);

FIG. 16C shows the SEM imaging result (Example 2-1) for circular M13mp18 double-stranded DNA (7.2 kb) molecules stained using mNG-HMG and PVP in combination, followed by stretching/immobilizing and imaging, along with the corresponding FM imaging result;

FIGS. 17A and 17B respectively show the SEM imaging result (Example 2-2) of genomic DNA molecules extracted from HeLa cells-stained using mNG-HMG and PVP in combination, then stretched/immobilized and imaged-and the corresponding FM imaging result, and FIG. 17C shows an SEM imaging result illustrating a Christmas-tree-like DNA structure potentially representing transcription along the backbone of genomic DNA derived from HeLa cells;

FIGS. 18A and 18B respectively show the SEM imaging result (Example 2-2) for mitochondrial DNA (16.6 kb) extracted from HeLa cells-stained using mNG-HMG and PVP in combination, then stretched/immobilized and imaged-and the corresponding FM imaging result;

FIG. 19A shows the result of FM imaging performed after immobilizing metaphase chromosomes extracted from HeLa cells onto a positively charged, surface-modified silicon wafer and staining them with the YOYO-1 staining reagent;

FIG. 19B shows the result of SEM imaging performed after immobilizing metaphase chromosomes extracted from HeLa cells onto a positively charged, surface-modified silicon wafer, and then staining only with PVP without DNA-binding protein;

FIG. 19C shows the result of SEM imaging performed after immobilizing metaphase chromosomes extracted from HeLa cells onto a positively charged, surface-modified silicon wafer, without staining by any DNA-binding protein or PVP, thereby observing the chromosomes in their native state;

FIG. 20A is a schematic diagram illustrating DNA stained using mNG-HMG (DBP-FP) and PVP (shown as black coils), and labeled with streptavidin-coated quantum dots, and FIGS. 20B and 20C respectively show enlarged views of QD-labeled sites from an SEM-based optical map of bacteriophage λ DNA generated in Example 3-2, and an enlarged view of the QD-labeled sites from the corresponding FM-based optical map;

FIG. 21A is a schematic diagram illustrating that circular M13mp18 double-stranded DNA (7.2 kb) contains two nick sites at 1373 bp and 1417 bp, and that DNA polymerase proceeds in two opposite directions, and FIGS. 21B and 21C respectively show the results of observing QD labeling in an SEM-based optical map for circular M13mp18 double-stranded DNA generated in Example 3-1, and the corresponding QD labeling observed in the FM-based optical map;

FIG. 22 compares the SEM-based DNA optical mapping result of bacteriophage λ DNA generated in Example 3-2 and the corresponding FM-based optical mapping result with an *in silico* map indicating the predicted labeling positions when Nb.BssSI is used for nick translation and QD labeling, based on the bacteriophage λ DNA sequence;

FIG. 23 shows enlarged images of all labeling sites in the SEM-based DNA optical mapping result of bacteriophage λ DNA generated in Example 3-2; and

FIG. 24 shows the SEM-based DNA optical mapping result for genomic DNA derived from HeLa cells, generated in Example 3-3.

## DETAILED DESCRIPTION

Reference will now be made in detail to embodiments, examples of which are illustrated in the accompanying

drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of", when preceding a list of elements, modify the entire list of elements and do not modify the individual elements of the list.

The following examples are provided to illustrate the present disclosure in further detail. However, these examples are for illustrative purposes only and should not be construed as limiting the scope of the present disclosure.

**[Reference Example]**

Preparation of DNA-Binding Proteins

**(1) mNeonGreen-HMG**

The mNeonGreen-HMG plasmid was constructed as described in "21 Fluorescent Protein-Based DNA Staining Dyes. Molecules 2022, 27(16)".

The DNA binding motif of high mobility group (HMG: TPKRPRGRPKK) was fused to the C-terminal region of mNeonGreen (mNG) provided by Dorus Gadella (Addgene plasmid # 98882) using polymerase chain reaction (PCR). The complete mNG-HMG was generated through overlap extension PCR. The amplified product was digested with restriction enzymes NdeI (CA^TATG) and BamHI (G^GATCC). The digested product was inserted into a pET15b vector. The amino acid sequence of mNeonGreen-HMG is shown in Table 1 below.

The constructed mNeonGreen-HMG plasmid was transformed into E. coli BL21(DE3) strain. BL21 cells harboring the mNG-HMG plasmid were incubated overnight at 37 °C in LB medium supplemented with ampicillin. Subsequently, 1 mL of the overnight culture was transferred to 100 mL of LB medium containing ampicillin and incubated at 37 °C until the optical density at 600 nm (OD600) reached 0.4-0.6. Protein expression was then induced by adding 1 mM IPTG, followed by overnight incubation at 20 °C to 25 °C with shaking at 200 rpm. Following sonication for 15 minutes and centrifugation at 12,000 × g for 10 minutes, mNG-HMG was harvested through affinity chromatography using Ni-NTA agarose resin. The mNG-HMG was eluted with a buffer containing 50 mM $Na_2HPO_4$, 300 mM NaCl, and 250 mM imidazole at pH 8.0. The purified protein was buffer-exchanged into PBS (phosphate-buffered saline: 137 mM NaCl, 2.7 mM KCl, 10 mM $Na_2HPO_4$, and 1.8 mM $KH_2PO_4$, pH 7.4), supplemented with glycerol, and stored at -20 °C.

**(2) tTALE-emGFP**

The tTALE-emGFP plasmid was constructed as described in "Truncated TALE-FP as DNA Staining Dye in a High-salt Buffer. Sci Rep-Uk 9, 17197 (2019)".

The tTALE-emGFP plasmid was generated through extension PCR by connecting the emGFP fluorescent protein to the C-terminal region of the DNA-binding protein TALE. The bridging sequence was GGSGG. The constructed tTALE-emGFP plasmid was transformed into E. coli BL21(DE3) strain using standard cloning procedures. The amino acid sequence of tTALE-emGFP is shown in Table 1 below.

**(3) H-NS**

The H-NS plasmid was constructed by amplifying the H-NS gene from H-NS-mCherry as described in Analyst 144, 921-927 (2019). The plasmid pET15b was digested with restriction enzymes NdeI (CA^TATG) and BamHI (G^GATCC). Subsequently, the H-NS gene was inserted and ligated into the plasmid using the AccuRapid cloning kit to generate the H-NS plasmid. The constructed H-NS plasmid was transformed into E. coli BL21(DE3) strain using standard cloning procedures. The amino acid sequence of H-NS is shown in Table 1 below.

**(4) H-NS-mScarlet**

The H-NS-mScarlet plasmid was constructed as described in "AT-specific DNA visualization revisits the directionality of bacteriophage lambda DNA ejection. Nucleic Acids Research (2023)".

The H-NS plasmid was digested with BamHI (G^GATCC). Subsequently, the amplified mScarlet gene was inserted and ligated into the H-NS plasmid using the AccuRapid cloning kit. The constructed H-NS-mScarlet plasmid was transformed into E. coli BL21(DE3) strain using standard cloning procedures. The amino acid sequence of H-NS-mScarlet is shown in Table 1 below.

**(5) Cro-mNeonGreen**

The Cro-mNeonGreen plasmid was constructed as described in "Negative nanopore sequencing for mapping biochemical processes on DNA molecules. Chemical Communications 59, 9388-9391 (2023)".

The Cro-mNeonGreen plasmid was generated by inserting the DNA-binding protein coding gene Cro into the mNeonGreen plasmid using a cassette vector system. The mNeonGreen plasmid was generated by inserting the mNeonGreen gene between the NdeI (CA^TATG) and XmaI (C^CCGGG) restriction sites in the pET15b vector. The Cro gene was amplified by PCR using bacteriophage λ DNA as a template. The constructed Cro-mNeonGreen plasmid was transformed into E. coli BL21(DE3) strain using standard cloning procedures. The amino acid sequence of Cro-mNeonGreen is shown in Table 1 below.

[Table 1]

| Name | Amino acid sequence | SEQ ID NO. |
|---|---|---|
| mNeonGree n-HMG | MGSSHHHHHHSSGLVPRGSHMMLPGCMLMVSKGEEDNMASLPATHELHI FGSINGVDFDMVGQGTGNPNDGYEELNLKSTKGDLQFSPWILVPHIGYGF HQYLPYPDGMSPFQAAMVDGSGYQVHRTMQFEDGASLTVNYRYTYEGSH IKGEAQVKGTGFPADGPVMTNSLTAADWCRSKKTYPNDKTIISTFKWSYTT GNGKRYRSTARTTYTFAKPMAANYLKNQPMYVFRKTELKHSKTELNFKEW QKAFTDVMGMDELYKLETPKRPRGRPKKMLGSGC (31.79 kDa) | 1 |
| Truncated TA-LE(t-TALE )-emGFP | MGSSHHHHHHSSGLVPRGSHMDLRTLGYSQQQQEKIKPKVRSTVAQHHE ALVGHGFTHAHIVALSQHPAALGTVAVKYQDMIAALPEATHEAIVGVGKQW SGARALEALLTVAGELRGPPLQLDTGQLLKIAKRGGVTAVEAVHAWRNALT GAPLNLTPAQVVAIASNNGGKQALETVQRLLPVLCQDHGLTPAQVVAIASN GGGKQALETVQRLLPVLCQAHGLTPDQVVAIASHDGGKQALETVQRLLPVL CQDHGLTPAQVVAIASNGGGKQALETVQRLLPVLCQAHGLTPDQVVAIASN NGGKQALETVQRLLPVLCQAHGLTPAQVVAIASNGGGKQALETVQRLLPVL CQDHGGGSGGMVSKGEELFTGVVPILVELDGDVNGHKFSVSGEGEGDAT YGKLTLKFICTTGKLPVPWPTLVTTFAYGLQCFARYPDHMKQHDFFKSAMP EGYVQERTISFKDDGNYKTRAEVKFEGDTLVNRIELKGTDFKEDGNILGHKL EYNYNSHNVYITADKQKNGIKANFKIRHNIEDGSVQLADHYQQNTPIGDGPV LLPDNHYLSTQSALSKDPNEKRDHMVLLEFVTAAGITLGMDELYK (64.7 kDa) | 2 |
| H-NS | MGSSHHHHHHSSGLVPRGSHMMSEALKILNNIRTLRAQARECTLETLEEML EKLEVVVNERREEESAAAAEVEERTRKLQQYREMLIADGIDPNELLNSLAA VKSGTKAKRAQRPAKYSYVDENGETKTWTGQGRTPAVIKKAMDEQGKSL DDFLIKQGSGC (18.10 kDa) | 3 |

(continued)

| Name | Amino acid sequence | SEQ ID NO. |
|---|---|---|
| H-NS-mScarlet | MGSSHHHHHHSSGLVPRGSHMMSEALKILNNIRTLRAQARECTLETLEEML EKLEVVVNERREEESAAAAEVEERTRKLQQYREMLIADGIDPNELLNSLAA VKSGTKAKRAQRPAKYSYVDENGETKTWTGQGRTPAVIKKAMDEQGKSL DDFLIKQEFMVSKGEAVIKEFMRFKVHMEGSMNGHEFEIEGEGEGRPYEG TQTAKLKVTKGGPLPFSWDILSPQFMYGSRAFTKHPADIPDYYKQSFPEGF KWERVMNFEDGGAVTVTQDTSLEDGTLIYKVKLRGTNFPPDGPVMQKKTM GWEASTERLYPEDGVLKGDIKMALRLKDGGRYLADFKTTYKAKKPVQMPG AYNVDRKLDITSHNEDYTVVEQYERSEGRHSTGGMDELYKGSGC (44.75 kDa) | 4 |
| CromNeonGreen | MGSSHHHHHHSSGLVPRGSHMMEQRITLKDYAMRFGQTKTAKDLGVYQS AINKAIHAGRKIFLTINADGSVYAEEVKPFPSNKKTTAGGSGGPGMVSKGEE DNMASLPATHELHIFGSINGVDFDMVGQGTGNPNDGYEELNLKSTKGDLQ FSPWILVPHIGYGFHQYLPYPDGMSPFQAAMVDGSGYQVHRTMQFEDGA SLTVNYRYTYEGSHIKGEAQVKGTGFPADGPVMTNSLTAADWCRSKKTYP NDKTIISTFKWSYTTGNGKRYRSTARTTYTFAKPMAANYLKNQPMYVFRKT ELKHSKTELNFKEWQKAFTDVMGMDELYKGSGC (37.07 kDa) | 5 |

Preparation of Substrates

Silicon wafers, silicon wafers with 30 nm-thick $SiO_2$ layer, and platinum-coated silicon wafers were purchased from Wafer Market (Yongin, Korea). Positively charged surface-modified silicon wafers were prepared by applying established methods for glass coverslips to silicon wafers with $SiO_2$ layer. Specifically, silicon wafers with $SiO_2$ layer were placed in a Teflon rack and immersed in piranha etching solution (30:70 v/v $H_2O_2/H_2SO_4$) for 3 hours. The wafers were thoroughly washed with deionized water, and pH 7 paper was used to confirm neutral pH. Subsequently, the wafers were sonicated in deionized water for 30 minutes and rinsed again with deionized water to expose the piranha surface. To prepare a 1.1 mM solution, 150 μL of Q-siloxane (N-trimethoxysilylpropyl-N,N,N-trimethyl ammonium chloride (Gelest)) in 50 % methanol was added to 250 mL deionized water. The wafers were incubated in 250 mL of the solution containing trimethoxysilylpropyl-N,N,N-trimethyl ammonium chloride (1.1 mM) at 65 °C with stirring at 100 rpm for 16 hours. Subsequently, the wafers were rinsed three times with 99.9 % ethanol and stored in 99.9 % ethanol to produce silicon wafers surface-modified with cations.

Preparation of PDMS Microfluidic Device

The PDMS microfluidic device was fabricated as described in "Counting DNA molecules on a microchannel surface for quantitative analysis, Talanta 2023, 252: 123826".

The PDMS device was fabricated using standard soft-lithography replication techniques. First, a microchannel template was deposited in two layers on a silicon wafer using repeated photolithography procedures, following the protocol described in the Kayaku Advanced Materials SU-8 2000 datasheet. Subsequently, a 20 μm-thick layer of SU-8 2015 photoresist was applied to the silicon wafer using a spin coater (Midas System SPIN-1200D, Daejeon, Korea). The spin-coated wafer was exposed to 350 nm radiation through a mask using an aligner (Midas System MDA-400LJ, Daejeon, Korea). After baking and developing the wafer using SU-8 developer, the second layer was precisely patterned on the developed wafer using a maskless lithography system SmartPrint (SmartForce Technologies, La Tronche, France). SU-8 TF 6002 was used due to SmartPrint's compatibility with g-line photoresist. Following template wafer fabrication, the microchannel template located on the silicon wafer was coated with a mixture of PDMS prepolymer and curing agent (10:1 weight ratio) and incubated at 65 °C for 12 hours. After peeling the cured PDMS from the wafer, chambers were created by

physically punching channels into the PDMS. Subsequently, the PDMS microchannels were oxidized using an air plasma generator (Femto Science Cute Basic, Korea) at 100 W for 30 seconds. Finally, the PDMS device was cleaned and stored in deionized water to produce the PDMS microfluidic device as shown in FIG. 2 (100 μm × 2.4 μm).

Preparation of DNA Samples

Bacteriophage λ DNA (48.5 kb) and circular M13mp18 DNA (7.2 kb) purchased from New England Biolabs were used. Genomic DNA, mitochondrial DNA, and metaphase chromosomes from HeLa cells were extracted using the following method:

(1) HeLa cells were cultured in DMEM (high glucose, pyruvate) medium supplemented with 10 % FBS (fetal bovine serum) and 1X antibiotic-antimycotic (Gibco). When HeLa cells reached approximately 80% confluency in the cell culture dish, the medium was carefully aspirated using a Pasteur pipette. Subsequently, 5 mL of 1X PBS buffer (phosphate-buffered saline: 137 mM NaCl, 2.7 mM KCl, 10 mM $Na_2HPO_4$, and 1.8 mM $KH_2PO_4$, pH 7.4) was slowly added to the side of the dish and aspirated. Next, 2 mL of trypsin-EDTA buffer was added and the dish was manually agitated to ensure coverage of the bottom surface. After incubating the cells for 2 minutes, 8 mL of medium was added, and the entire solution (10 mL) was transferred to a 15 mL conical tube and centrifuged at 500 g for 10 minutes. The HeLa cells were then resuspended in 1 mL of 1X PBS buffer and transferred to an Eppendorf tube. After washing twice with 1X PBS buffer, the cells were finally resuspended in 500 μL of 1X PBS buffer.

(2) Genomic DNA was prepared in agarose gel plugs. The cell suspension was mixed with 2 % 1X TE LGT solution to achieve a final LGT concentration of 0.7 % and incubated at 4 °C for 30 minutes. The DNA plugs were washed in 1X TE buffer for 30 minutes, and this step was repeated 4 times. Proteinase K was added to a final concentration of 2 mg/mL, and the mixture was incubated at 42 °C for 4 hours. An equal amount of Proteinase K was added again, followed by overnight incubation at 42 °C. The DNA plugs were then washed in 1X TE buffer for 30 minutes, and this step was repeated 4 times. Finally, 380 μL of 1X TE buffer was added, and the DNA plugs were melted by incubating at 65 °C for 20 minutes.

(3) Mitochondrial DNA was prepared according to the QIAprep Spin Miniprep Kit protocol (Qiagen). The cell suspension was first centrifuged at 1,000 g for 10 minutes. After discarding the supernatant, cells were resuspended in 300 μL of P1 buffer. Subsequently, 300 μL of P2 buffer was added, followed by 420 μL of N3 buffer. The tube was then centrifuged at 12,000 g for 10 minutes. The resulting supernatant was transferred to a QIAprep spin column and centrifuged at 12,000 g for 1 minute. The plasmid was eluted with 100 μL of elution buffer. The volume of the eluted solution was measured precisely, and AMPure XP beads were added at 0.4X the measured solution volume. The solution was mixed well for 5 minutes to ensure DNA binding to the beads. Using a magnetic separation stand, the beads were allowed to adhere to the magnet for 1 minute. The solution was slowly removed from the opposite side of the magnet-bound beads, and 200 μL of 80 % ethanol was added. The stand was gently inverted 5 times and the ethanol was removed. This process was repeated twice. Residual ethanol was removed by air-drying the tube with the lid open at room temperature for approximately 5 minutes. The tube was removed from the stand, and 25 μL of 0.1X TE buffer was added and mixed well with the beads. After standing at room temperature for 10 minutes, the beads were allowed to adhere to the magnet using the magnetic separation stand for 1 minute. Mitochondrial DNA was isolated by transferring the solution to another tube.

(4) Metaphase chromosomes were prepared as follows: 100 μL of colcemid stock solution was added to the DMEM medium containing cultured HeLa cells to achieve a final concentration of 0.01 mg/mL. After overnight incubation in a cell culture incubator, the culture medium was collected in a 15 mL centrifuge tube. Subsequently, 2 mL of Trypsin-EDTA buffer was dispensed onto the cells, manually agitated to cover the dish bottom, and incubated for 2 minutes. Then, 5 mL of medium was added and the entire solution was transferred to the same 15 mL centrifuge tube. After centrifugation at 100 g for 5 minutes, the supernatant was removed and cells were resuspended in the remaining medium. Slowly, 5 mL of 75 mM KCl solution was added along the tube wall, and cells were incubated in this solution for 15 minutes. Subsequently, 5 mL of freshly prepared fixative (methanol:glacial acetic acid 3:1) was slowly added and mixed by inverting the tube. After centrifugation at 200 g for 5 minutes, the supernatant was removed and cells were resuspended in 10 mL of fixative. Finally, the previous step was repeated 4 times and cells were resuspended in 200 μL of fixative.

**[EXAMPLES]**

**Example 1-1. SEM Imaging of DNA Using DNA-Binding Protein and Polymer (mNG-HMG and PVP)**

25 pg of bacteriophage λ DNA (48.5 kb, New England Biolabs) was mixed with 792 nM mNeonGreen-high mobility group (mNG-HMG, 31 kDa) and incubated for 10 minutes, then diluted with 1 × TE buffer solution (Tris 10 mM EDTA 1 mM pH 8.0), after which an equal volume of a 5 % polyvinylpyrrolidone (PVP, 40 kDa, Sigma-Aldrich) solution was added and incubated for 5 minutes to prepare the DNA solution.

1 μL of the prepared DNA solution was injected into a polydimethylsiloxane (PDMS) microfluidic device mounted on the surface of a positively charged, surface-modified silicon wafer (coated with 1.1 mM Q-siloxane), which guided the solution through a microchannel (see FIG. 2, indicated by the arrow). Before separating the PDMS microfluidic device from the positively charged, surface-modified silicon wafer, the solution within the microchannel was dried for 50 minutes, thereby stretching and immobilizing the DNA onto the positively charged, surface-modified silicon wafer. Afterward, the PDMS microfluidic device was separated from the silicon wafer surface, and the DNA was imaged with a scanning electron microscope (SEM, JEOL, JSM-7100F) at 15 kV and magnifications of X 5000 to X 50,000. For image processing, ImageJ software was used to measure the resolution of the DNA image by employing the 'doFWHM' macro.

**Example 1-2. SEM Imaging of DNA Using DNA-Binding Protein and Polymer (H-NS and PVP)**

An SEM DNA image was obtained through the same staining, stretching/immobilizing, and imaging procedures as in Example 1-1, except that 264 nM histone-like nucleoid structuring protein (H-NS, 18 kDa) was used instead of 792 nM mNG-HMG.

**Example 1-3. SEM Imaging of DNA Using DNA-Binding Protein and Polymer (H-NS-mScarlet and PVP)**

An SEM DNA image was obtained through the same staining, stretching/immobilizing, and imaging procedures as in Example 1-1, except that 264 nM histone-like nucleoid structuring protein-mScarlet (H-NS-mScarlet, 45 kDa) was used instead of 792 nM mNG-HMG.

**Example 1-4. SEM Imaging of DNA Using DNA-Binding Protein and Polymer (tTALE-EmGFP and PVP)**

An SEM DNA image was obtained through the same staining, stretching/immobilizing, and imaging procedures as in Example 1-1, except that 396 nM truncated TALE-EmGFP (tTALE-EmGFP, 65 kDa) was used instead of 792 nM mNG-HMG.

**Example 1-5. SEM Imaging of DNA Using DNA-Binding Protein and Polymer (Cro-mNeonGreen and PVP)**

An SEM DNA image was obtained through the same staining, stretching/immobilizing, and imaging procedures as in Example 1-1, except that 264 nM Cro-mNeonGreen (37 kDa) was used instead of 792 nM mNG-HMG.

**Example 1-6. SEM Imaging of DNA Using DNA-Binding Protein and Polymer (mNG-HMG and PEOX)**

An SEM DNA image was obtained through the same staining, stretching/immobilizing, and imaging procedures as in Example 1-1, except that 132 nM mNG-HMG was used instead of 792 nM mNG-HMG, and a 1 % poly(2-ethyl-2-oxazoline) (PEOX, 50 kDa, Sigma-Aldrich) aqueous solution was used instead of the 5 % PVP aqueous solution.

**Example 1-7. SEM Imaging of DNA Using DNA-Binding Protein and Polymer (mNG-HMG and PANI)**

An SEM DNA image was obtained through the same staining, stretching/immobilizing, and imaging procedures as in Example 1-1, except that 396 nM mNG-HMG was used instead of 792 nM mNG-HMG, and a 0.1 % polyaniline (PANI, 40 kDa, Sigma-Aldrich) aqueous solution was used instead of the 5 % PVP aqueous solution.

**Example 2-1. SEM Imaging of Circular M13mp18 DNA (7.2 kb) Sample**

An SEM DNA image was obtained through the same staining, stretching/immobilizing, and imaging procedures as in Example 1-1, except that circular M13mp18 DNA (7.2 kb) was used as the DNA sample instead of bacteriophage λ DNA.

**Example 2-2. SEM Imaging of HeLa Cell-Derived Genomic DNA Sample**

An SEM DNA image was obtained through the same staining, stretching/immobilizing, and imaging procedures as in Example 1-1, except that the genomic DNA extracted from HeLa cells according to Reference Example (2) was used as the DNA sample instead of bacteriophage λ DNA.

**Example 2-3. SEM Imaging of HeLa Cell-Derived Mitochondrial DNA Sample**

An SEM DNA image was obtained through the same staining, stretching/immobilizing, and imaging procedures as in Example 1-1, except that mitochondrial DNA extracted from HeLa cells according to Reference Example (3) was used as the DNA sample instead of bacteriophage λ DNA.

**Example 3-1. SEM-Based DNA Optical Mapping via QD Labeling**

Nick translation was performed after nickase treatment to label the DNA sample with quantum dots. Specifically, a total of 500 ng of M13mp18 double-stranded DNA was incubated at 16 °C for 2 hours in NEB buffer 2 with 20 units of Nb.BbvCI (New England Biolabs), 10 units of DNA polymerase I (New England Biolabs), and a nucleotide mixture containing biotin-labeled dUTP (Biotin-16-dUTP, Jena Bioscience), dTTP, dATP, dCTP, and dGTP. After the nick translation, the DNA solution was diluted to a final concentration of 10 ng/μL. The resulting solution was then mixed with 30 nM streptavidin-conjugated quantum dots (Qdot 585, Thermo Fisher Scientific) and incubated at room temperature for 10 minutes. In order to separate DNA from unbound Qdot 585, a 25 nm mixed-cellulose-ester membrane was placed over 100 μL of 1 × TE buffer, and the DNA solution was applied to the membrane and allowed to stand and be incubated for 30 minutes. Then, the DNA solution on the membrane was carefully recovered. The recovered DNA solution, following labeling, was stained, stretched/immobilized, and imaged in the same manner as in Example 1-1, thereby generating an SEM-based optical map.

**Example 3-2. SEM-Based DNA Optical Mapping of Bacteriophage** Λ **DNA Sample**

An SEM-based optical map was generated through the same labeling, staining, stretching/immobilizing, and imaging procedures as in Example 3-1, except that bacteriophage λ DNA (48.5 kb, New England Biolabs) was used as the DNA sample instead of circular M13mp18 DNA, and Nb.BssSI (New England Biolabs), which cleaves at the 5'-C^TCGTG-3' site, was used instead of Nb.BbvCI.

**Example 3-3. SEM-Based DNA Optical Mapping of HeLa Cell-Derived Genomic DNA Sample**

An SEM-based optical map was generated through the same labeling, staining, stretching/immobilizing, and imaging procedures as in Example 3-1, except that the genomic DNA extracted from HeLa cells according to Reference Example (2) was used as the DNA sample instead of circular M13mp18 DNA, and Nb.BssSI (New England Biolabs), which cleaves at the 5'-C^TCGTG-3' site, was used instead of Nb.BbvCI.

**[COMPARATIVE EXAMPLES]**

**Comparative Example 1-1. SEM Imaging of DNA Using DNA-Binding Protein (mNG-HMG)**

An SEM DNA image was obtained in the same staining, stretching/immobilizing, and imaging procedures as in Example 1-1, except that a DNA solution prepared by mixing 25 pg of bacteriophage λ DNA (48.5 kb, New England Biolabs) with 792 nM mNeonGreen-HMG and incubating for 10 minutes was used.

**Comparative Example 1-2. SEM Imaging of DNA Using Polymer (PVP)**

An SEM DNA image was obtained in the same staining, stretching/immobilizing, and imaging procedures as in Example 1-1, except that a DNA solution prepared by mixing 25 pg of bacteriophage λ DNA (48.5 kb, New England Biolabs) with a 5 % PVP (40 kDa) solution and incubating for 10 minutes was used.

**Comparative Example 1-3. SEM Imaging of DNA Using Polymer (PANI)**

An SEM DNA image was obtained in the same staining, stretching/immobilizing, and imaging procedures as in Example 1-1, except that a DNA solution prepared by mixing 25 pg of bacteriophage λ DNA (48.5 kb, New England

Biolabs) with a 0.1 % polyaniline (PANI, 40 kDa) aqueous solution and incubating for 10 minutes was used, and the DNA solution was loaded onto a positively charged, surface-modified silicon wafer without using a microfluidic device.

## Experimental Example 1. Evaluation of DNA Stainability in SEM According to Various Staining Reagents

In order to determine whether the DNA-binding protein-polymer complexes can function as staining reagents that visualize DNA under scanning electron microscopy (SEM), the degree of DNA staining was compared and evaluated using SEM images of bacteriophage λ DNA obtained in Examples 1-1 to 1-7 and Comparative Examples 1-1 to 1-3. As an untreated control, a DNA solution prepared by diluting 1 ng of λ phage DNA molecules (48.5 kb) in 1 × TE (Tris 10 mM, EDTA 1 mM, pH 8.0) buffer was stretched/immobilized and imaged according to the same method as in Example 1-1, and the resulting SEM image was used. As a control, the imaging results obtained by fluorescence microscopy (FM) instead of SEM were used. Unless otherwise stated hereinafter, the FM images were acquired by the method described in Lee et al., Nucleic Acids Research, Volume 44, Issue 1 (2016).

FIG. 3 shows an SEM imaging result (untreated control) of bacteriophage λ DNA molecules obtained by stretching/immobilizing and imaging in the same manner as in Example 1-1, without any staining reagent treatment.

FIG. 4 shows an SEM imaging result (Comparative Example 1-1) for bacteriophage λ DNA molecules stained using mNG-HMG alone, followed by stretching/immobilization and imaging, along with the corresponding FM imaging result.

FIG. 5 shows an SEM imaging result (Comparative Example 1-2) for bacteriophage λ DNA molecules stained using PVP alone, and then stretched/immobilized and imaged.

FIG. 6 shows an SEM imaging result (Example 1-2) for bacteriophage λ DNA molecules stained using H-NS and PVP in combination, followed by stretching/immobilizing and imaging.

FIG. 7 shows an SEM imaging result (Example 1-3) for bacteriophage λ DNA molecules stained using H-NS-mScarlet and PVP in combination, followed by stretching/immobilizing and imaging, along with the corresponding FM imaging result.

FIG. 8 shows an SEM imaging result (Example 1-4) for bacteriophage λ DNA molecules stained using tTALE-EmGFP and PVP in combination, followed by stretching/immobilizing and imaging, along with the corresponding FM imaging result.

FIG. 9 shows an SEM imaging result (Example 1-5) for bacteriophage λ DNA molecules stained using Cro-mNeonGreen and PVP in combination, followed by stretching/immobilizing and imaging, along with the corresponding FM imaging result.

FIG. 10 shows an SEM imaging result (Example 1-6) for bacteriophage λ DNA molecules stained using mNG-HMG and poly(2-ethyl-2-oxazoline) in combination, followed by stretching/immobilizing and imaging, as well as an SEM imaging result for bacteriophage λ DNA molecules stained using mNG-HMG and poly(2-ethyl-2-oxazoline) in combination, and then immobilized on a positively charged, surface-modified silicon wafer without using a microfluidic device, and imaged.

FIG. 11 shows an SEM imaging result (Example 1-7) for bacteriophage λ DNA molecules stained using mNG-HMG and polyaniline in combination, followed by stretching/immobilizing and imaging.

FIG. 12 shows an SEM imaging result (Comparative Example 1-3) for bacteriophage λ DNA molecules stained using polyaniline alone, and then immobilized on a positively charged, surface-modified silicon wafer without using a microfluidic device.

FIG. 15 shows an SEM imaging result (Example 1-1) for bacteriophage λ DNA molecules stained using mNG-HMG and PVP in combination, followed by stretching/immobilizing and imaging.

As shown in FIGS. 3 to 11, DNA molecules without any staining reagent (untreated control), those stained using mNG-HMG alone (Comparative Example 1-1), or with PVP alone (Comparative Example 1-2) were difficult to visualize under SEM. In contrast, when a DNA-binding protein or a DNA-binding protein-fluorescent protein (such as H-NS, H-NS-mScarlet, tTALE-EmGFP, or Cro-mNeonGreen) was used in combination with PVP (Examples 1-2 to 1-5), DNA appeared as a dark line under SEM, similarly to the result obtained when mNG-HMG and PVP were used (Example 1-1, FIG. 15). This indicates that successful SEM staining was achieved. Additionally, DNA was also successfully stained when other polymers, such as poly(2-ethyl-2-oxazoline) or polyaniline, were employed (Examples 1-6 to 1-7).

As shown in FIG. 12, however, the image of DNA stained using polyaniline alone (Comparative Example 1-3) formed large aggregates containing branched DNA. Thus, although polyaniline can bind DNA without protein assistance, the image quality significantly deteriorates.

These findings demonstrate that effective electrostaining of DNA molecules for SEM can be achieved by utilizing a DNA-binding protein in combination with a polymer capable of binding thereto.

Experimental Example 2. Evaluation of SEM Image Contrast Depending on Substrate Surface Characteristics

In order to assess how the surface characteristics of the substrate used to immobilize DNA molecules affect contrast in SEM images, droplets of a PVP solution were placed on four different surfaces (a positively charged, surface-modified silicon wafer, a silicon wafer with an $SiO_2$ layer, a plain silicon wafer, and a platinum-coated silicon wafer) then dried, and the edges of each droplet were captured by SEM. The results are shown in FIG. 13.

FIG. 13 illustrates PVP droplets placed on various functionalized silicon wafer surfaces and visualized by SEM. Specifically, a positively charged, surface-modified silicon wafer, a silicon wafer with an $SiO_2$ layer, a plain silicon wafer, and a platinum-coated silicon wafer were used. The dashed line in the platinum-coated wafer image indicates the droplet boundary of the PVP solution.

As shown in FIG. 13, the SEM snapshots of the PVP droplet edges on the silicon wafer surfaces (positively charged, surface-modified silicon wafer, silicon wafer with an $SiO_2$ layer, or plain silicon wafer) revealed a distinct boundary between the dark PVP region and the adjacent wafer area. However, on the platinum-coated wafer surface, the droplet boundary indicated by the dashed line was faint and difficult to discern. This indicates that, on a semiconductor surface such as a silicon wafer, PVP droplets appear as dark shapes because they interfere with secondary electron emission. Conversely, on a fully conductive surface, such as a platinum-coated wafer, the greatly increased passage and emission of secondary electrons through the surface PVP layer diminish the contrast at the droplet boundary. Accordingly, when DNA is visualized under SEM using PVP, employing a semiconductor silicon wafer surface can further enhance background contrast. Moreover, because an $SiO_2$ layer can immobilize the DNA backbone when derivatized with a quaternary ammonium siloxane, subsequent experiments were conducted using a positively charged, surface-modified silicon wafer surface.

**Experimental Example 3. Evaluation of DNA Extension with or without Microfluidic Device**

In order to assess the effect of using a microfluidic device on supplying DNA onto a positively charged, surface-modified silicon wafer, a DNA solution containing a staining reagent was either introduced into a microfluidic device or simply loaded onto the substrate without such a device, and SEM images were then evaluated. Bacteriophage λ DNA (48.5 kb, New England Biolabs) was used as the DNA sample.

Specifically, an SEM image was obtained in the same manner as in Example 1-1, except that the DNA solution containing the staining reagent was applied to a positively charged, surface-modified silicon wafer and dried, without using a microfluidic device for the extension/immobilization step. For the control group, imaging results acquired by fluorescence microscopy (FM) instead of SEM were used. FIGS. 14A and 14B present the results. The results are shown in FIGS. 14A and 14B. When a microfluidic device was used, the staining, stretching/immobilizing, and imaging were carried out as in Example 1-1, and the resulting SEM image is shown in FIG. 15.

FIGS. 14A and 14B respectively show the SEM imaging result and the corresponding FM imaging result for bacteriophage λ DNA molecules stained using mNG-HMG and PVP in combination, and then applied and immobilized onto the surface of a positively charged, surface-modified silicon wafer without using a microfluidic device, and imaged.

FIG. 15 shows the SEM imaging result (Example 1-1) of bacteriophage λ DNA molecules stained using mNG-HMG and PVP in combination, followed by stretching/immobilizing and imaging.

As shown in FIGS. 14A and 14B, even without using a microfluidic device, the SEM image revealed more intricate fine structures of the DNA compared to the FM image. Furthermore, as shown in FIGS. 14A and 15, the DNA exhibited a random arrangement when no microfluidic device was used, whereas the DNA appeared in a parallel, elongated configuration when a microfluidic device was employed. This indicates that the DNA was guided and stretched by the microchannel of the microfluidic device, and became immobilized on the positively charged surface of the $SiO_2$ layer.

**Experimental Example 4. Evaluation of Resolution in Stained DNA Images**

In order to evaluate the resolution of SEM-based DNA imaging using a staining reagent according to an embodiment of the present disclosure, the thickness of DNA in SEM images and FM images was compared using samples of bacteriophage λ DNA (48.5 kb) and circular M13mp18 DNA (7.2 kb).

Specifically, the SEM DNA images of bacteriophage λ DNA (48.5 kb) and circular M13mp18 DNA (7.2 kb) were obtained according to Examples 1-1 and 2-1, respectively. For the control group, the DNA thickness was observed in images obtained by fluorescence microscopy (FM) instead of SEM. The results are shown in FIGS. 16A and 16C. In addition, to accurately measure DNA thickness, the "doFWHM" ImageJ macro (downloaded from GitHub) was used to compute the full width at half maximum (FWHM) of intensity profiles derived from inverted SEM DNA images, and FIG. 16B shows the results of those computations.

FIG. 16A illustrates the SEM imaging result (Example 1-1) for bacteriophage λ DNA (48.5 kb) stained using mNG-HMG and PVP in combination, followed by stretching/immobilizing and imaging, along with the corresponding FM imaging

result. FIG. 16B shows an enlarged, inverted version of the SEM imaging result for the λ DNA (48.5 kb) in FIG. 16A, together with the intensity profile along the yellow line of the inverted SEM DNA image and the measured FWHM value.

FIG. 16C shows the SEM imaging result (Example 2-1) for circular M13mp18 double-stranded DNA (7.2 kb) stained using mNG-HMG and PVP in combination, followed by stretching/immobilizing and imaging, along with the corresponding FM imaging result.

As shown in FIG. 16A, although the length of λ DNA remained constant in both the SEM and FM images, a substantial difference in thickness was observed. Specifically, compared to the FM image, the SEM image exhibited a finer, continuous black line at higher resolution, with respective thickness values of approximately 15 nm versus 450 nm, indicating a significant disparity.

Furthermore, as shown in FIG. 16B, the FWHM of the SEM DNA image was on average 15.0 ± 4.0 nm (ranging from 9.4 nm to 22.8 nm), whereas the FWHM of the FM DNA image was on average 239 ± 30 nm. The measured FWHM in the inverted SEM DNA image was 9.4 nm, consistent with the observation from the inverted SEM image.

The FWHM calculation was based on the assumption that the DNA molecule has a thickness of 2 nm, the mNeonGreen-HMG protein has a diameter of 2.4 nm and a height of 4.2 nm, and the PVP polymer has a weight-average molecular weight (Mw) of 40 kDa and a radius of gyration (Rg) of 3.8 nm as reported in "Enhancement in Elastic Bending Rigidity of Polymer Loaded Reverse Microemulsion, Langmuir 2017, 33(45): 13014-13026". Under the assumption that the PVP polymer is circular, its outer diameter (d_out) is approximately 9.8 nm according to Equation 1 below. The assembly of protein and polymer surrounding the DNA molecule likely contributes to the observed average thickness of 15.0 ± 4.0 nm in FIG. 16B.

[Equation 1]

$$d_{out} = 2\sqrt{5/3}R_g$$

Also, as shown in FIG. 16C, the high-resolution SEM image vividly captured circular or supercoiled DNA structures, which appear only as small dots in the FM image.

These findings indicate that SEM-based DNA imaging using a staining reagent according to an embodiment provides a resolution of 15 nm, meaning the resolution of SEM is far superior to the approximately 239 nm resolution for DNA images obtained by FM.

## Experimental Example 5. Visualization of Various Types of DNA Molecules

In order to determine whether SEM-based DNA imaging using a staining reagent according to an embodiment can visualize DNA molecules derived from human cells, SEM images were evaluated for genomic DNA, mitochondrial DNA, and metaphase chromosomes extracted from HeLa cells.

Specifically, the SEM DNA images of HeLa cell-derived genomic DNA and mitochondrial DNA were obtained according to Examples 2-2 and 2-3, respectively. For the control group, images acquired by FM instead of SEM were used. The results are presented in FIGS. 17A to 17C, and FIGS. 18A and 18B.

Also, in order to observe metaphase chromosomes extracted from HeLa cells, 2 μL of the cell solution prepared according to Reference Example (4) was placed on a positively charged silicon wafer and dried. To remove the cell membrane, the silicon wafer was first immersed in distilled water for 15 minutes, then treated with 0.15 M phosphate buffer (pH 6) containing 0.0125 % trypsin for 1 minute. Next, the wafer was washed with distilled water and incubated in the same phosphate buffer at 37 °C for 1 hour. Finally, after further incubation in distilled water for 3 hours, the silicon wafer was immersed in either a 5 % PVP solution or a YOYO-1 solution. Subsequently, SEM or FM images were obtained. The results are shown in FIGS. 19A to 19C.

FIG. 17A and FIG. 17B respectively illustrate the SEM imaging result (Example 2-2) for genomic DNA molecules extracted from HeLa cells-stained using mNG-HMG and PVP in combination, then stretched/immobilized and imaged-and the corresponding FM imaging result. FIG. 17C shows the SEM imaging result for a Christmas-tree-like DNA structure potentially indicating transcription along the backbone of the HeLa cell-derived genomic DNA.

FIG. 18A and FIG. 18B respectively illustrate the SEM imaging result (Example 2-2) for mitochondrial DNA (16.6 kb) extracted from HeLa cells-stained using mNG-HMG and PVP in combination, then stretched/immobilized and imaged-and the corresponding FM imaging result.

FIG. 19A shows the result of FM imaging performed after immobilizing metaphase chromosomes extracted from HeLa cells on a positively charged, surface-modified silicon wafer and staining them with the YOYO-1 staining reagent.

FIG. 19B shows the result of SEM imaging performed after immobilizing metaphase chromosomes extracted from HeLa cells on a positively charged, surface-modified silicon wafer, and then staining only with PVP in the absence of a DNA-binding protein.

FIG. 19C shows the result of SEM imaging performed after immobilizing metaphase chromosomes extracted from HeLa cells on a positively charged, surface-modified silicon wafer, without staining by any DNA-binding protein or PVP, thus observing the native state of the chromosome.

As shown in FIG. 17B, the FM image appears thick and occasionally tangled, making it difficult to ascertain the structure, and there is a considerable variation in the fluorescence intensity of the DNA image. By contrast, as shown in FIG. 17A, the SEM image distinctly reveals the DNA and its spatial distribution. The SEM image can identify multiple DNA molecules in close proximity that cannot be distinguished by the FM image.

Moreover, as shown in FIG. 17C, a "Christmas-tree-like" structure indicative of chromatin transcription-which is reported to be unresolvable by FM due to the diffraction limit-was identified. Until now, electron microscopy images depicting such a structure have been reported in amphibian oocytes, insects, yeast, and mice, but not in human cells or cell lines. In FIG. 17C, the yellow arrow is presumed to indicate the DNA backbone, while the progressively denser, brush-shaped lines are presumed to represent transcribed RNA molecules.

As shown in FIG. 18B, despite a length of 16.6 kb, the mitochondrial DNA extracted from HeLa cells appeared simply as a dot in the FM image. However, as shown in FIG. 18A, the SEM image revealed the complex form of the circular mitochondrial DNA.

Moreover, as shown in FIG. 19A, given that a metaphase chromosome ranges from 5 μm to 10 μm in size, the metaphase chromosome could be readily observed under FM by staining with YOYO-1. In the case of SEM imaging, attempting to stain the metaphase chromosome with mNG-HMG and PVP resulted in a dark cluster image, which was unsuitable. Assuming that chromosomes contain abundant proteins, an attempt was made to observe the metaphase chromosome by SEM using only PVP without a DNA-binding protein. The chromosome in the native state without PVP was also observed by SEM. As shown in FIGS. 19B and 19C, SEM could provide high-resolution, magnified images of chromosomes even without staining. Thus, the metaphase chromosome can be observed on a silicon wafer with or without using PVP, although a chromosome coated with PVP appears darker due to PVP binding.

Experimental Example 6. Evaluation of Resolution in SEM-Based DNA Optical **Mapping**

Based on the confirmation that various types of DNA molecules can be visualized through SEM-based DNA imaging using a staining reagent according to an embodiment, an assessment was conducted with a variety of DNA samples to see if SEM-based optical DNA mapping can surpass the resolution provided by FM-based optical DNA mapping.

Specifically, as a model system, circular M13mp18 double-stranded DNA and Nb.BbvCI (New England Biolabs), which cleaves at the 5'-CC^TCAGC-3' site, were used to generate an SEM-based DNA optical map according to an embodiment (Example 3-1). For the control, an FM-based DNA optical map obtained by imaging Example 3-1 with FM instead of SEM was used. The results are shown in FIGS. 21B and 21C.

Additionally, as another model system, bacteriophage λ DNA and Nb.BssSI (New England Biolabs), which cleaves at the 5'-C^TCGTG-3' site, were used to generate an SEM-based DNA optical map according to an embodiment (Example 3-2). For the control, an FM-based DNA optical map obtained by imaging Example 3-2 with FM instead of SEM was used. The results are shown in FIGS. 20B, 20C, 22, and 23.

Additionally, as a model system, genomic DNA extracted from HeLa cells and Nb.BssSI (New England Biolabs), which cleaves at the 5'-C^TCGTG-3' site, were used to generate an SEM-based DNA optical map according to an embodiment (Example 3-3). The results are shown in FIG. 24.

FIG. 20A is a schematic diagram illustrating DNA stained using mNG-HMG (DBP-FP) and PVP (represented by black coils), and labeled with streptavidin-coated quantum dots. FIGS. 20B and 20C respectively show an enlarged view of the QD-labeled sites from the SEM-based DNA optical map of bacteriophage λ DNA generated in Example 3-2, and an enlarged view of the QD-labeled sites from the corresponding FM-based DNA optical map.

FIG. 21A is a schematic diagram indicating that circular M13mp18 double-stranded DNA (7.2 kb) contains two nick sites at positions 1373 bp and 1417 bp, and that DNA polymerase proceeds in two opposite directions. FIGS. 21B and 21C respectively show the results of observing QD labeling in the SEM-based DNA optical map for circular M13mp18 double-stranded DNA generated in Example 3-1, and the corresponding QD labeling in the FM-based DNA optical map.

FIG. 22 compares the SEM-based DNA optical mapping result and the corresponding FM-based DNA optical mapping result for bacteriophage λ DNA generated in Example 3-2 with an *in-silico* map that indicates the predicted labeling positions when Nb.BssSI is used for nick translation and QD labeling based on the sequence of bacteriophage λ DNA.

FIG. 23 shows enlarged images of all labeling sites from the SEM-based DNA optical mapping result of bacteriophage λ DNA generated in Example 3-2.

FIG. 24 shows the SEM-based DNA optical mapping result for genomic DNA derived from HeLa cells, generated in Example 3-3.

As shown in FIGS. 20B and 20C, in the SEM image, dozens of QDs were clearly distinguishable, whereas in the

FM image, those QDs merged into a single dot. Moreover, in FIGS. 21B and 21C, which capture circular DNA labeled with QDs, the FM image displayed merged signals from the green FP and red QDs as a single bright yellow spot; however, the individual SEM images revealed 20-30 QD labels clearly distributed along the circular DNA backbone.

Furthermore, as shown in FIG. 22, the *in-silico* map predicted a total of eight labeling sites. In the FM-based optical map, however, it was difficult to observe all eight sites due to the short distances between certain labeling sites-specifically, the fifth and sixth sites were 789 bp apart (789 bp × 0.34 nm/bp = 268 nm), and the seventh and eighth sites were 321 bp apart (321 bp × 0.34 nm/bp = 109 nm). In the FM-based optical map, the fifth and sixth sites merged into a single point (789 bp, 268 nm), and the seventh and eighth sites likewise merged into one point (321 bp, 109 nm). Consequently, only six red labeling sites could be distinguished in the FM-based optical map. In contrast, the SEM-based optical map successfully revealed all eight sites, with each site displaying five QDs as shown in the enlarged image of the seventh and eighth sites. Furthermore, as shown in FIG. 23, QD labeling was confirmed at all eight sites. Since each cluster, composed of multiple nanoparticles, precisely identifies the location of sequence-specific nick translation, these findings demonstrate that the SEM-based optical DNA mapping of the present disclosure achieves substantially higher resolution compared to FM-based optical DNA mapping.

Furthermore, as shown in FIG. 24, successful visualization of both the DNA backbone and QD-labeled regions was achieved using genomic DNA from HeLa cells, confirming the capability of the present disclosure's SEM-based optical DNA mapping to perform high-resolution optical mapping of human genomic DNA.

According to an aspect, the method of optical DNA mapping, by utilizing SEM as the basic platform, provides several advantages: enhanced accessibility and user-friendliness, superior compatibility with chemically functionalized surfaces and microfluidic devices, and the ability to obtain high-resolution DNA images without metal usage, thereby enabling optical DNA mapping with improved resolution.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the spirit and scope of the disclosure as defined by the following claims.

## Claims

**1.** A method of scanning electron microscopy (SEM)-based optical DNA mapping, the method comprising:

(a) contacting a double-stranded DNA with a nickase to form a nick at a target sequence motif;
(b) generating labeled double-stranded DNA by incorporating labeled nucleotides into a nick site of the nicked double-stranded DNA;
(c) staining a backbone of the labeled double-stranded DNA;
(d) stretching and immobilizing the stained double-stranded DNA on a substrate having a functionalized surface; and
(e) determining the distance between each label by imaging the immobilized double-stranded DNA using a scanning electron microscope (SEM).

**2.** The method of claim 1, wherein the nickase is Nb.BbvCl, Nb.BssSI, Nb.Bsml, Nb.BsrDI, Nb.Btsl, Nt.Alwl, Nt.BbvCl, Nt.BsmAl, Nt.BspQI, Nt.BstNBI, Nt.CviPll, nCAS9, or a combination thereof.

**3.** The method of claim 1, wherein the labeled nucleotide is a nucleotide to which a detectable label is conjugated either directly or indirectly.

**4.** The method of claim 3, wherein the detectable label is a nanoparticle or a fluorescent dye.

**5.** The method of claim 4, wherein the nanoparticle is any one selected from the group consisting of metal nanoparticles, oxide nanoparticles, sulfide nanoparticles, nanoclusters, quantum dots, graphene quantum dots, perovskite, carbon dots, polymer particles, hydroxyapatite, and magnetic nanoparticles.

**6.** The method of claim 1, wherein step (b) comprises:

contacting the nicked double-stranded DNA with a DNA polymerase in the presence of at least one first binding-moiety-conjugated nucleotide, to generate a modified double-stranded DNA having a first binding moiety; and

contacting the modified double-stranded DNA with a detectable label conjugated to a second binding moiety, to generate labeled double-stranded DNA,
wherein the second binding moiety forms a binding pair with the first binding moiety.

**7.** The method of claim 6, wherein the first binding moiety or the second binding moiety is any one selected from the group consisting of biotin, streptavidin, digoxin, neutravidin, and avidin.

**8.** The method of claim 6, wherein the DNA polymerase has 5'→3' exonuclease activity.

**9.** The method of claim 1, wherein step (c) comprises contacting the labeled double-stranded DNA with a DNA-binding protein.

**10.** The method of claim 9, wherein the DNA-binding protein comprises a fluorescent protein.

**11.** The method of claim 9, wherein step (c) further comprises contacting the double-stranded DNA with a polymer capable of binding to the DNA-binding protein, an anhydrate thereof, or a salt thereof.

**12.** The method of claim 11, wherein the polymer, the anhydrate thereof, or the salt thereof, comprises at least one structural unit represented by Formulas 1 to 9, and has a weight-average molecular weight (Mw) of 10 kDa to 100 kDa:

[Formula 1]

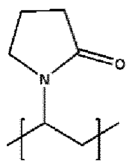

,

[Formula 2]

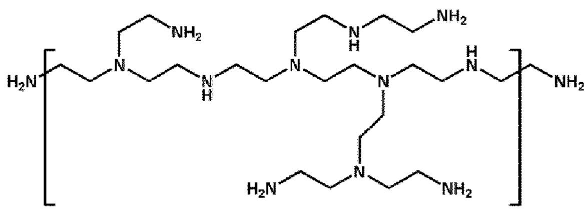

,

[Formula 3]

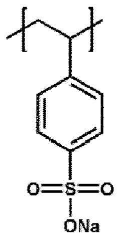

,

[Formula 4]

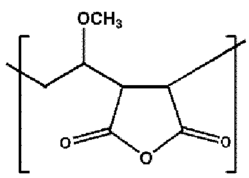

,

[Formula 5]

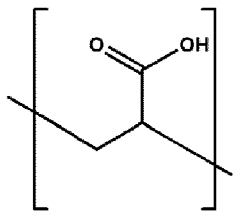

,

[Formula 6]

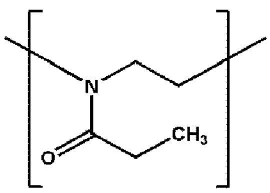

,

[Formula 7]

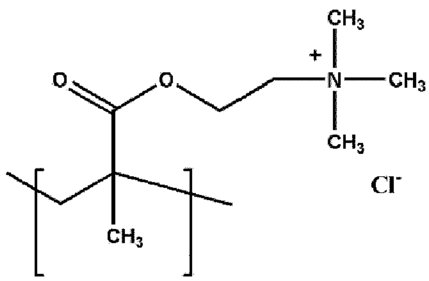

,

[Formula 8]

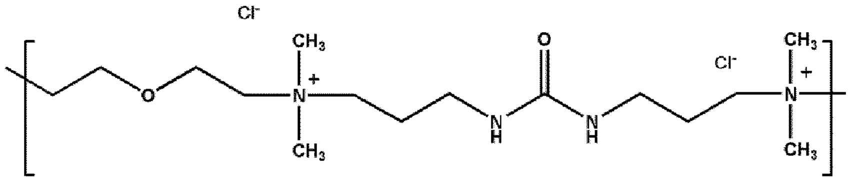

,

[Formula 9]

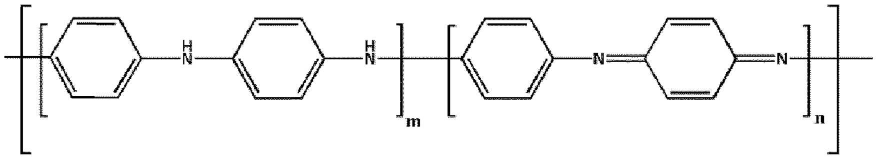

wherein in Formulas 1 to 9,
m + n is 1.

**13.** The method of claim 1, wherein step (c) is carried out in a metal-free manner.

**14.** The method of claim 1, wherein the substrate having a functionalized surface is a silicon substrate positively charged via surface modification.

**15.** The method of claim 1, wherein step (d) comprises passing the stained double-stranded DNA through a microchannel.

FIG. 1

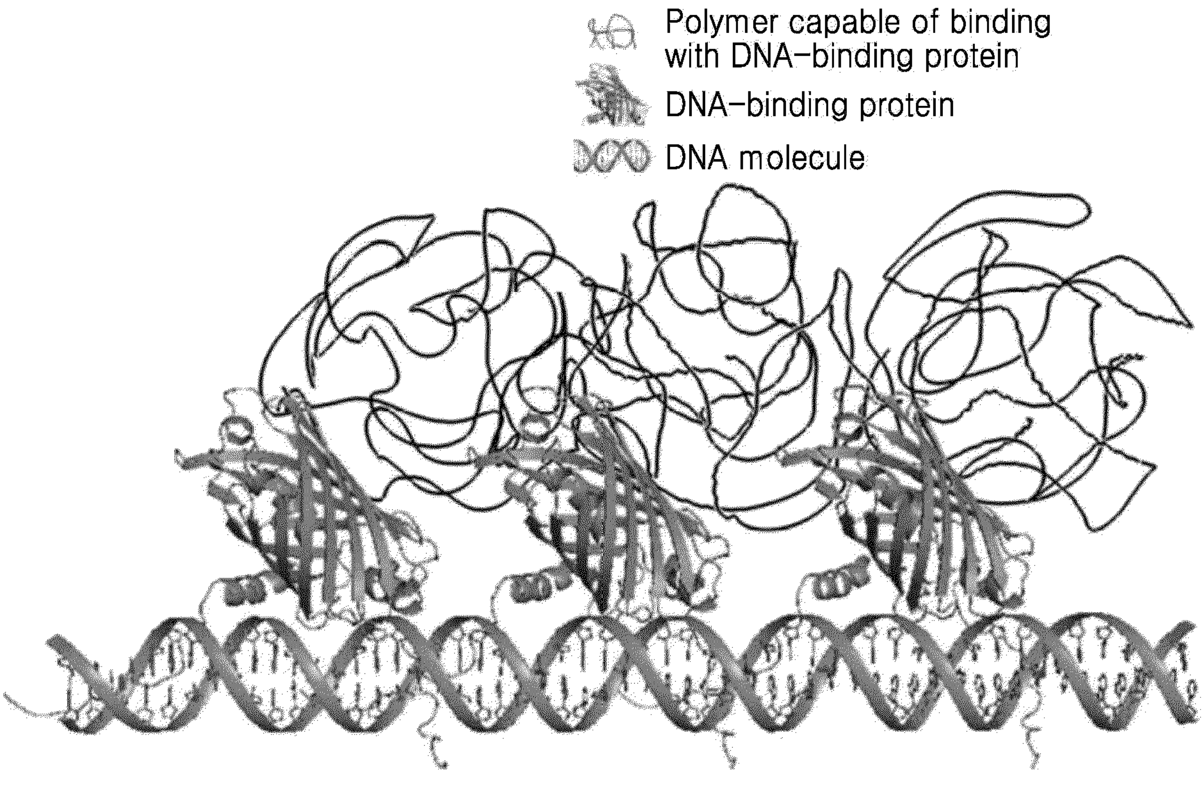
Polymer capable of binding
with DNA-binding protein
DNA-binding protein
DNA molecule

FIG. 2

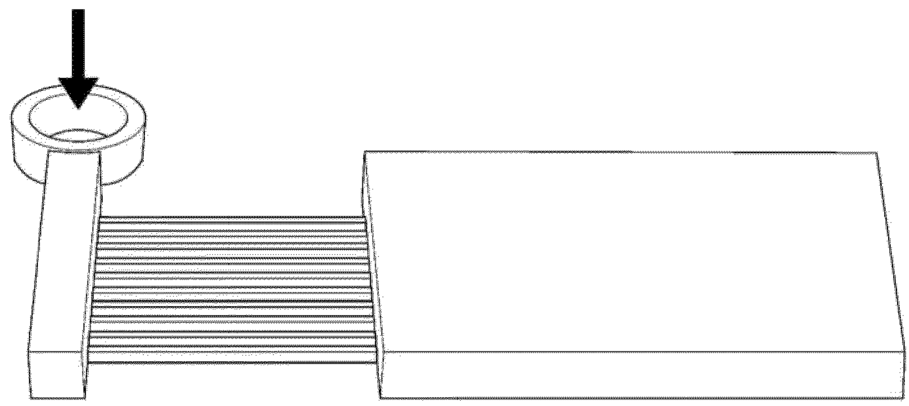

FIG. 3

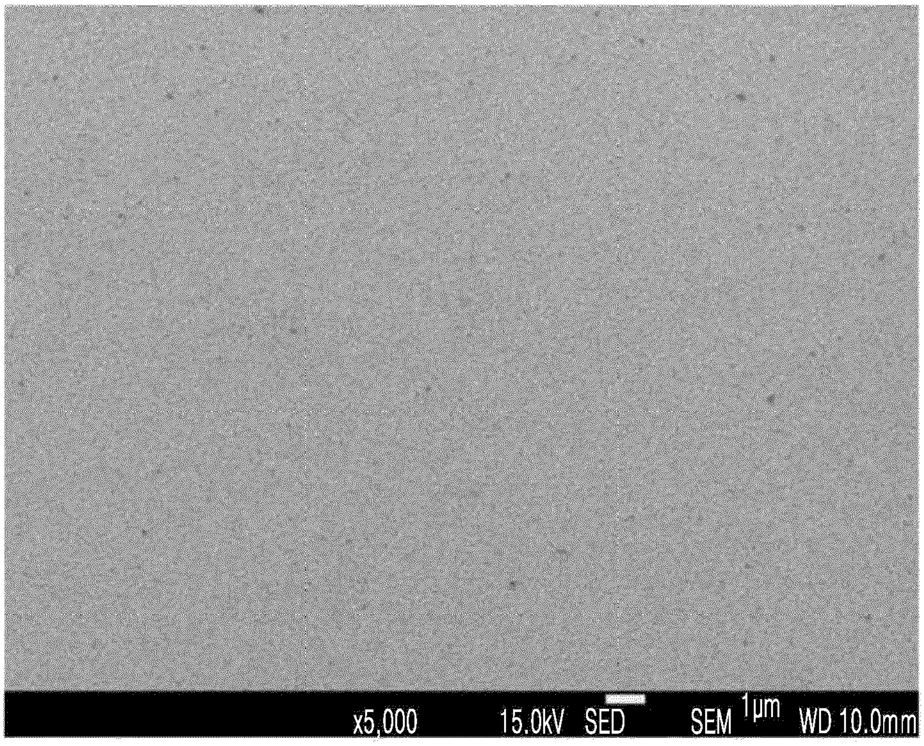


DNA only under SEM

FIG. 4

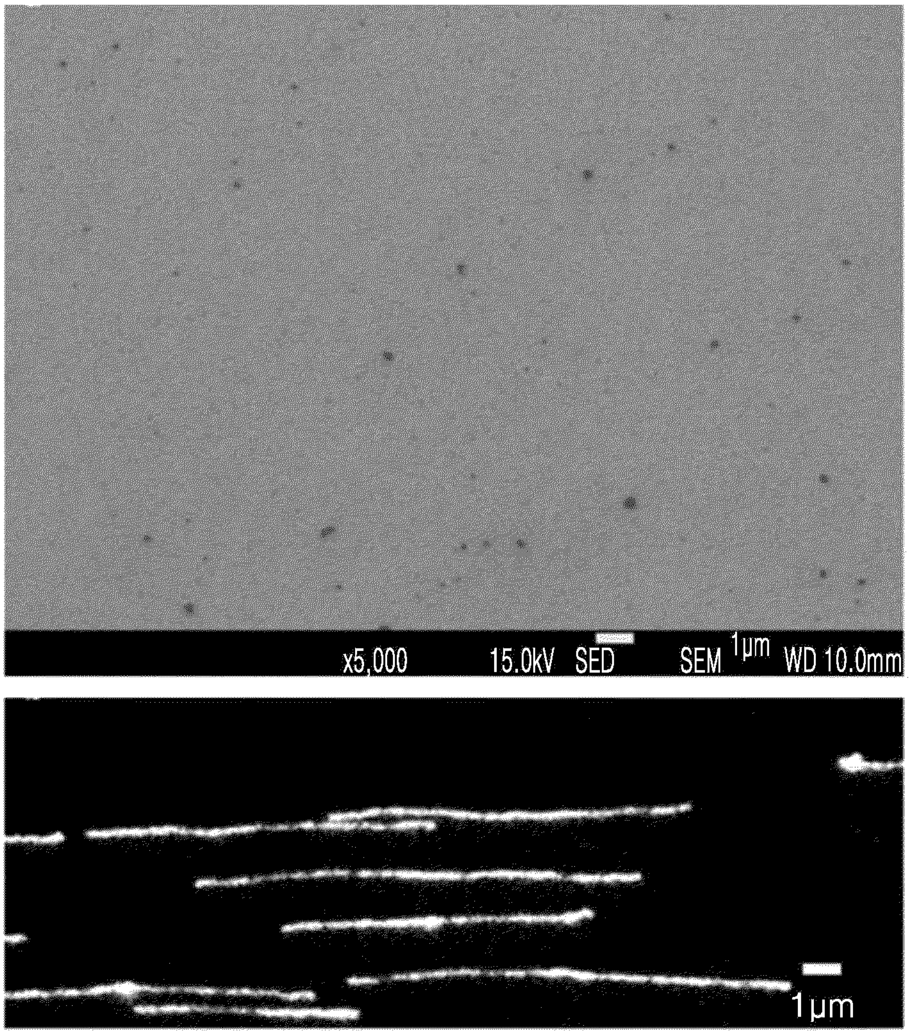


SEM

FM

mNeongreen-HMG stained DNA under SEM and FM

FIG. 5

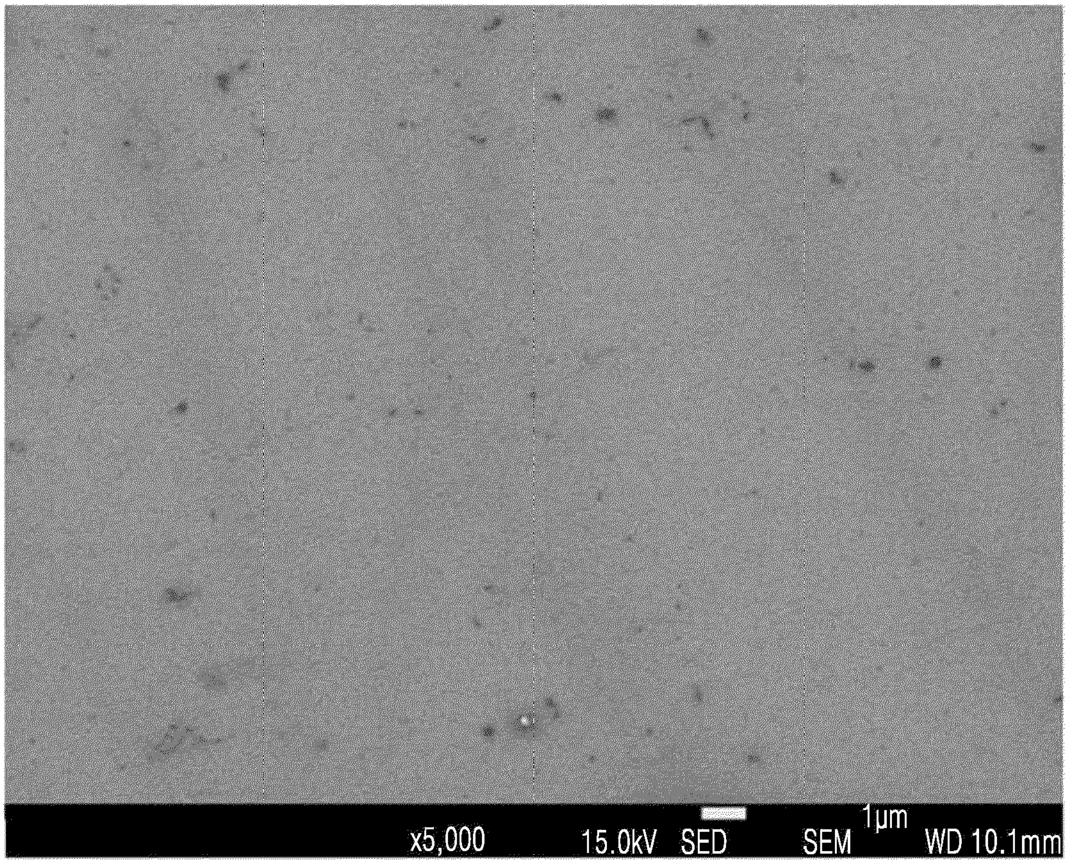


PVP and DNA mixture under SEM

FIG. 6

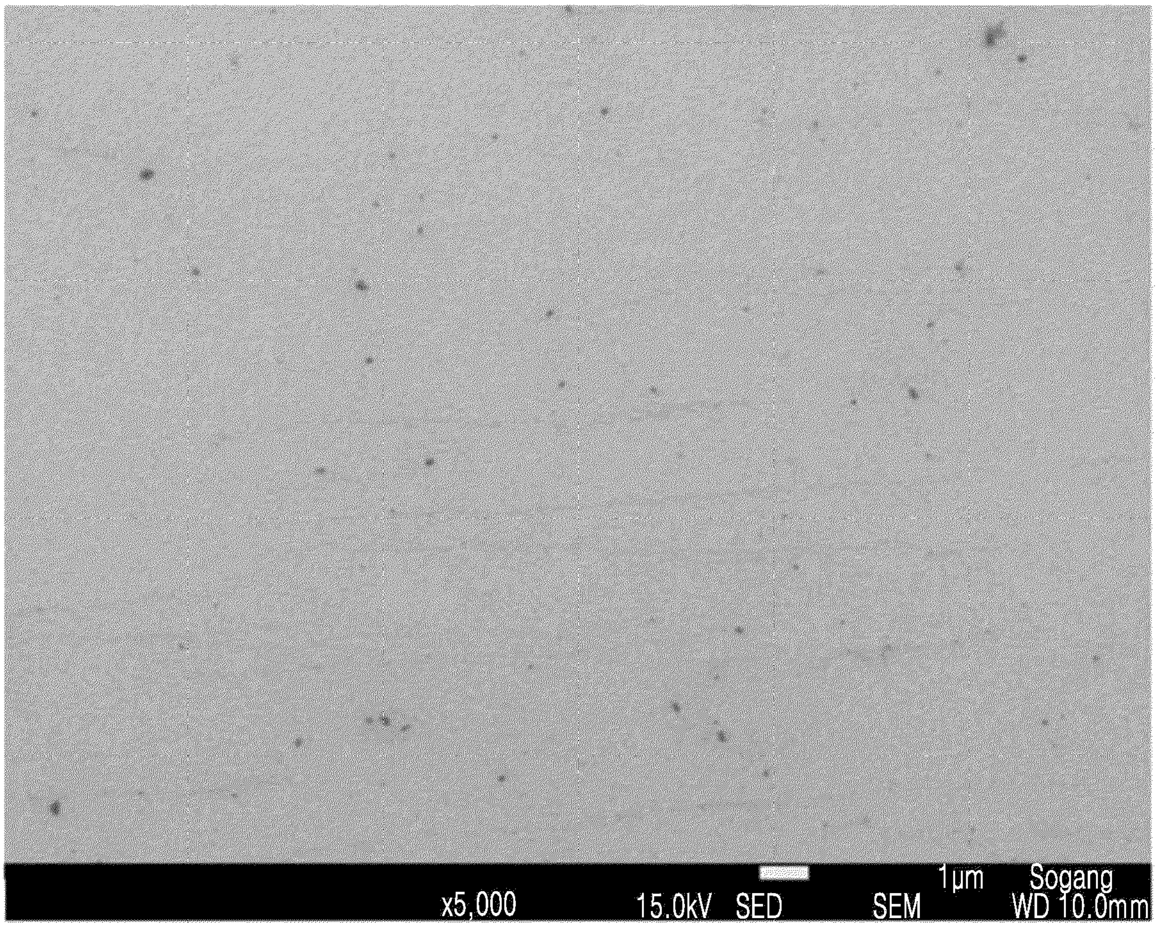


H-NS and PVP stained DNA under SEM

# FIG. 7

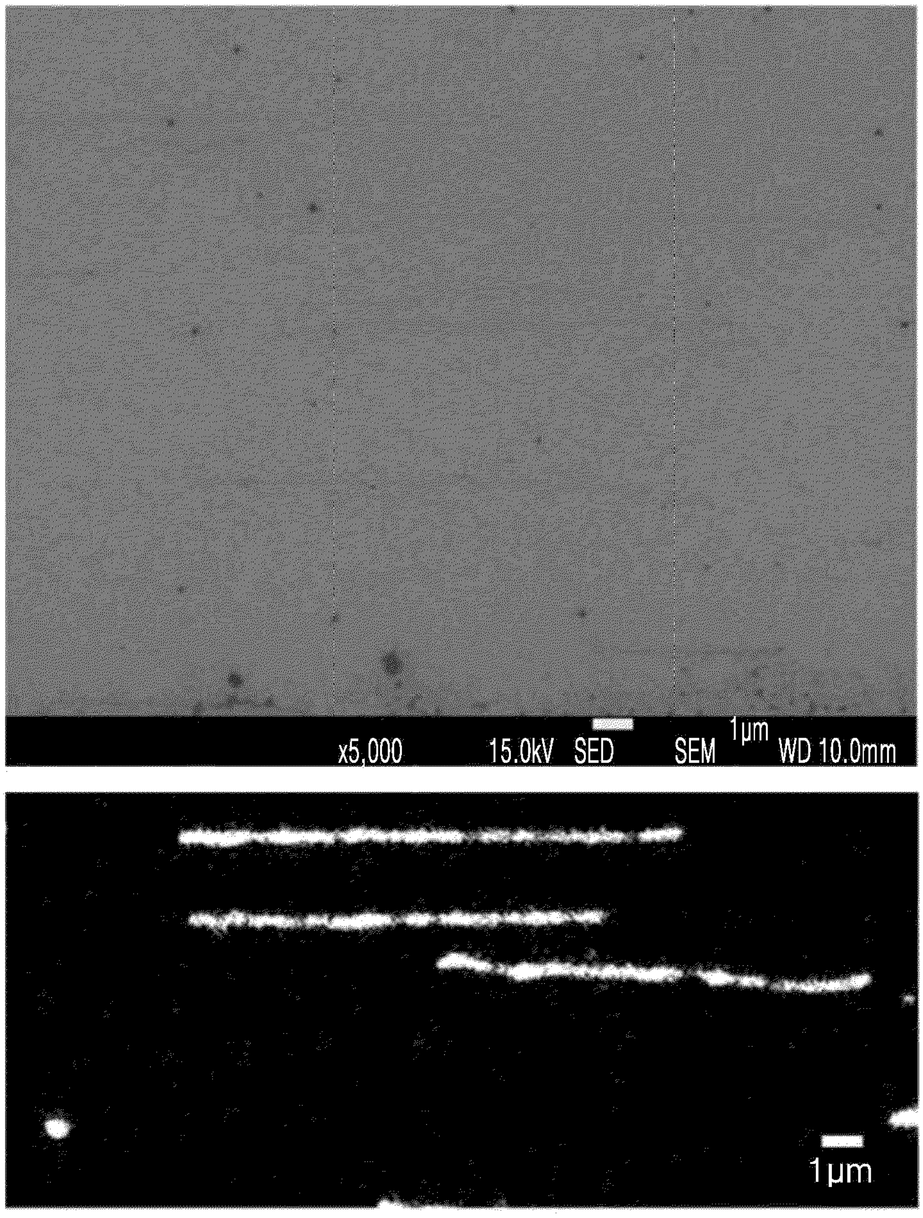


SEM

FM

H-NS-mScarlet and PVP stained DNA under SEM and FM

FIG. 8

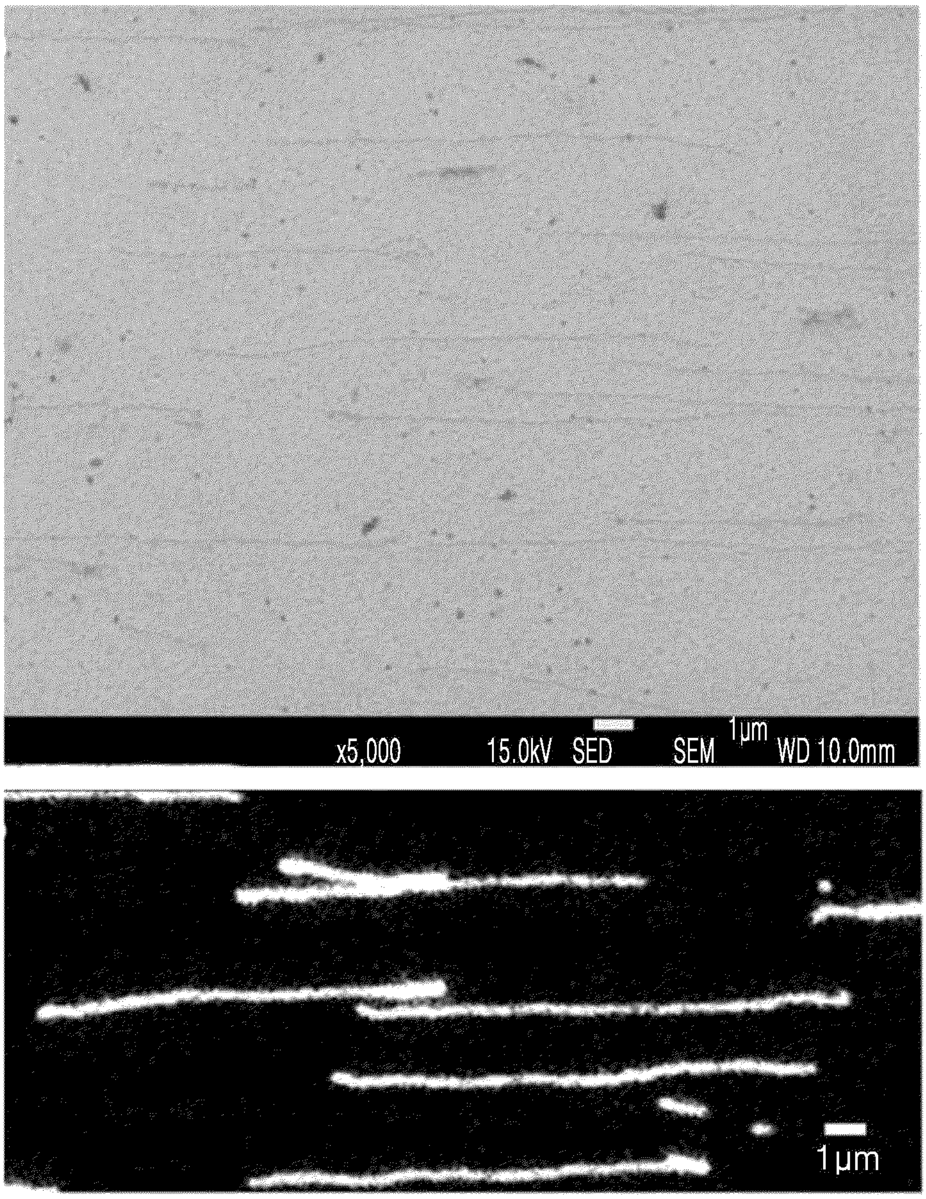


SEM

FM

tTALE-EmGFP and PVP stained DNA under SEM and FM

FIG. 9

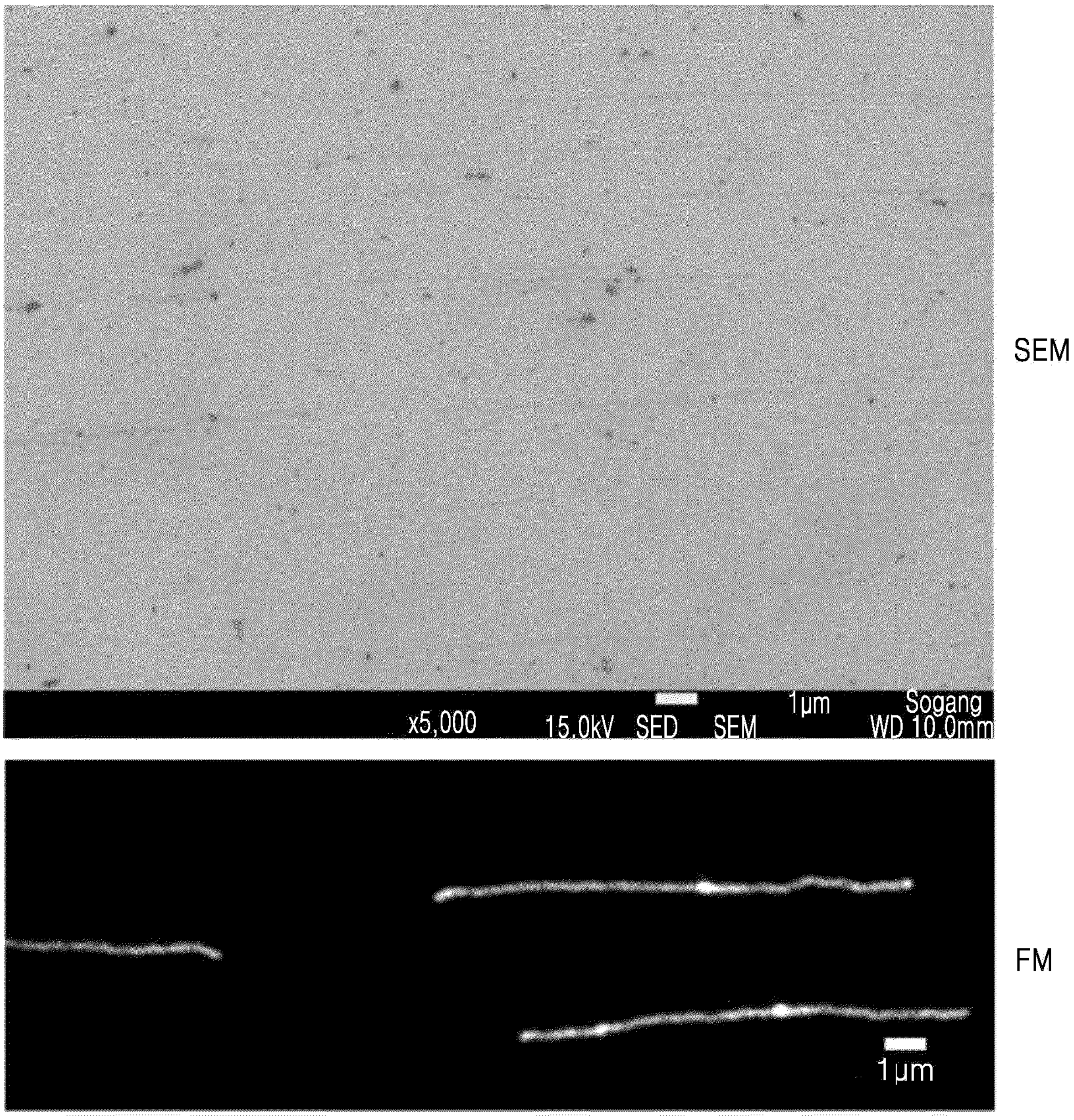


Cro-mNeongreen and PVP stained DNA under SEM and FM

# FIG. 10

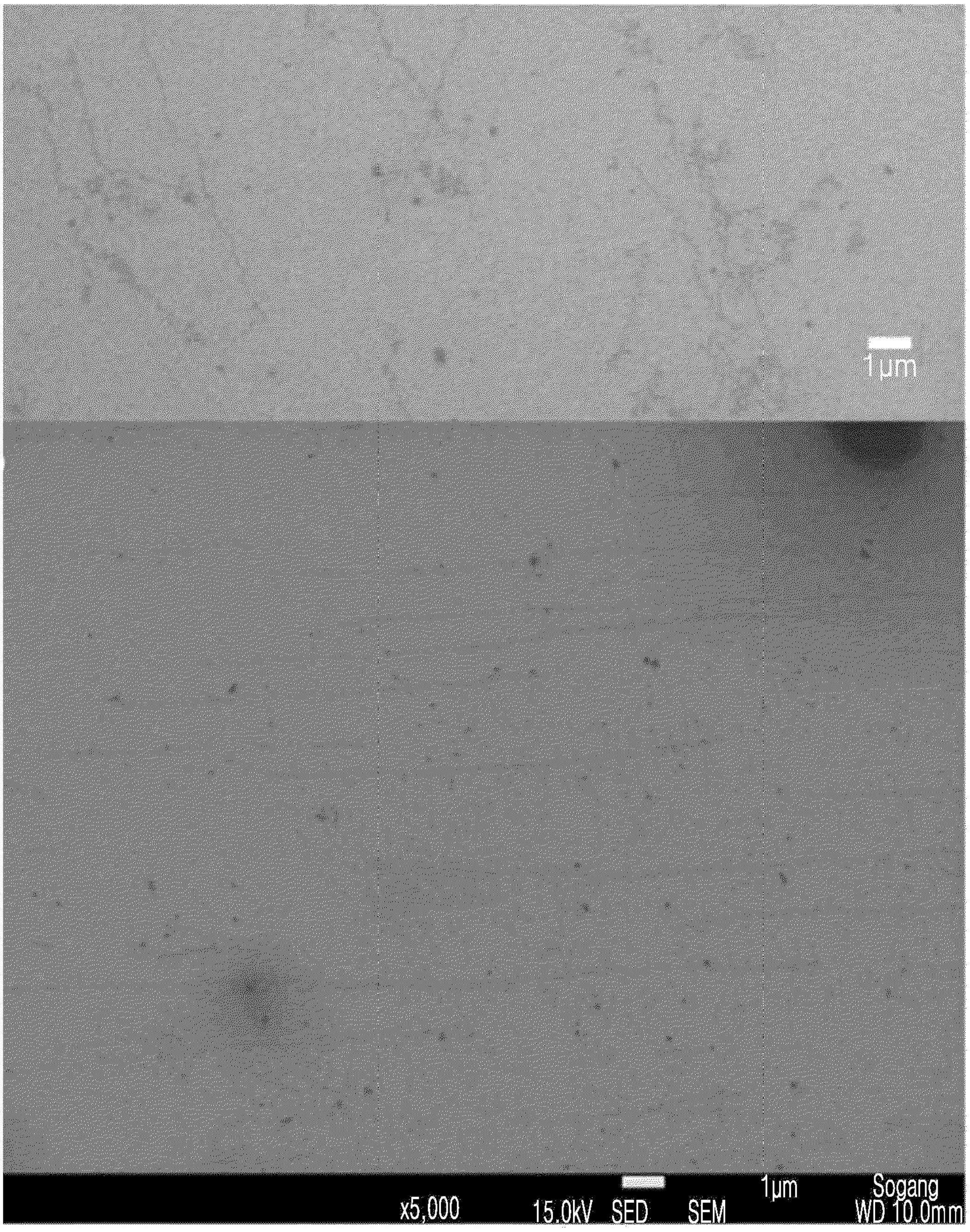


w/o microfluidic deivce

w/ microfluidic deivce

poly(2-ehtyl-2-oxazoline) and mNeongreen-HMG stained DNA under SEM

FIG. 11

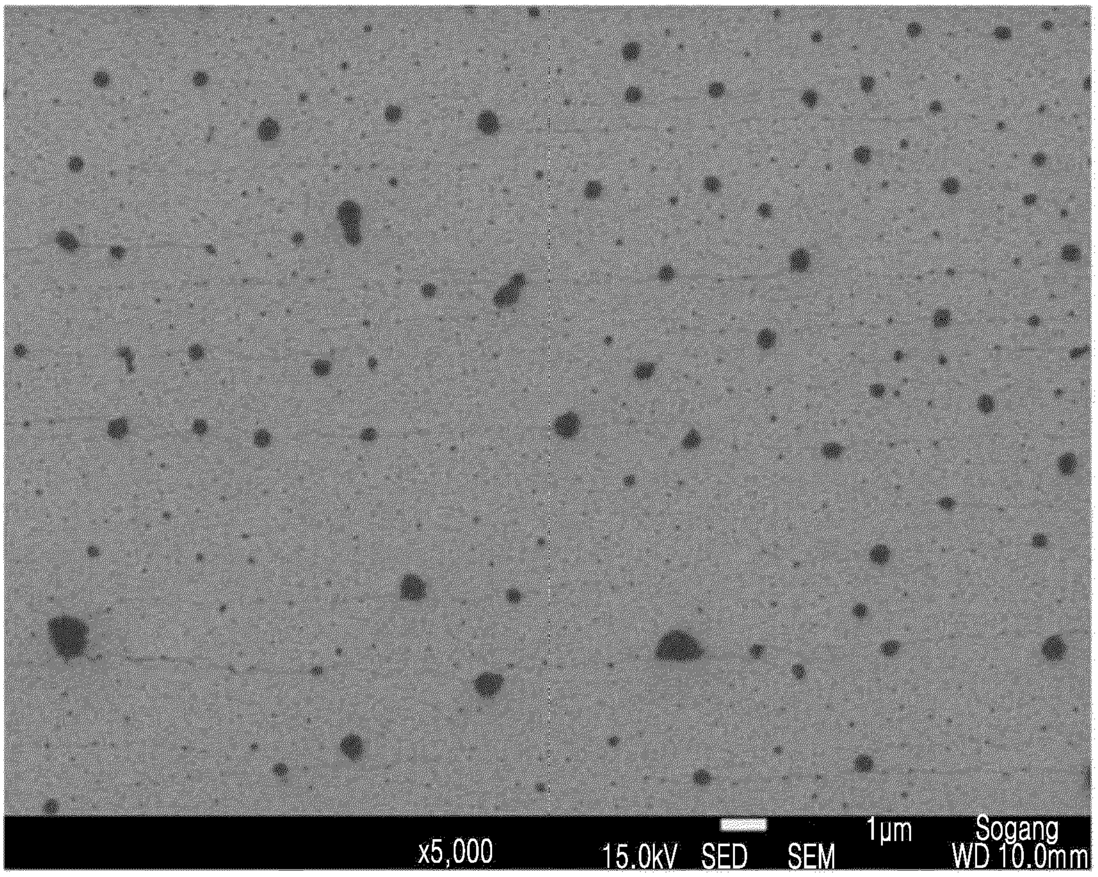


Polyaniline and mNeongreen-HMG stained DNA under SEM

FIG. 12

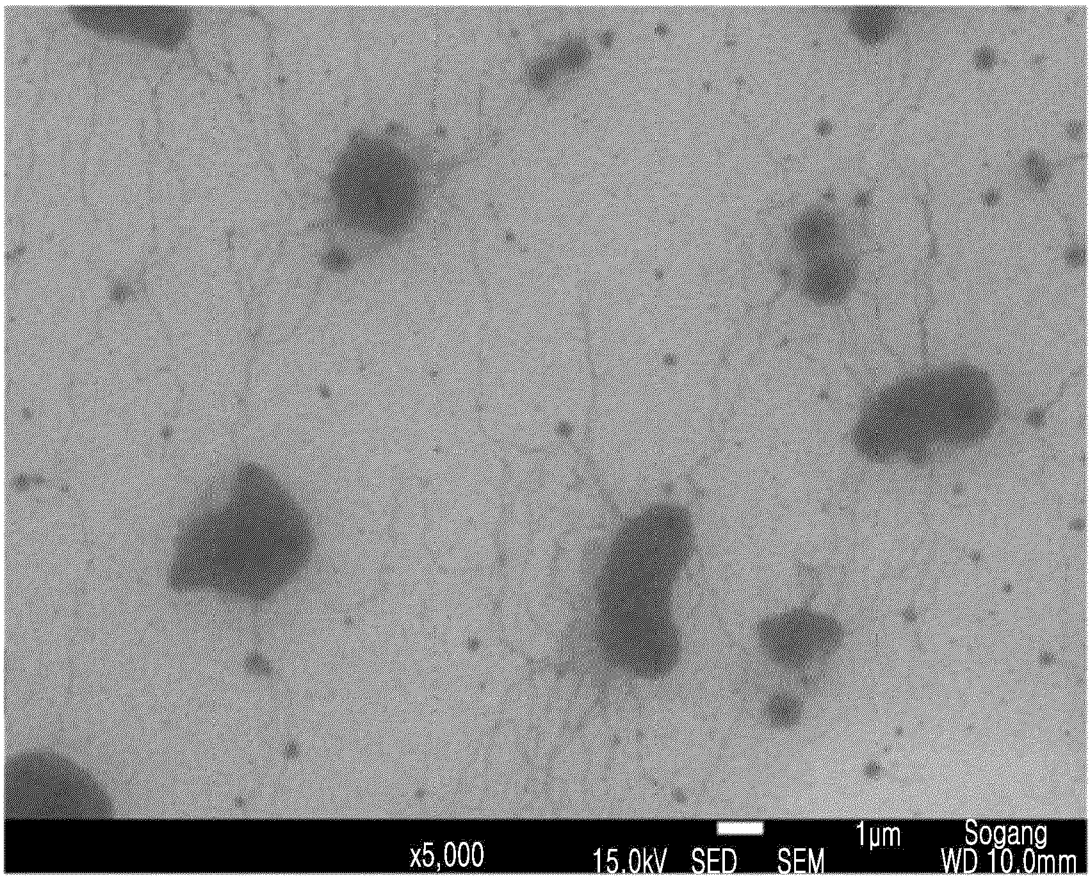


Polyaniline and DNA mixture under SEM (w/o microfluidic device)

FIG. 13

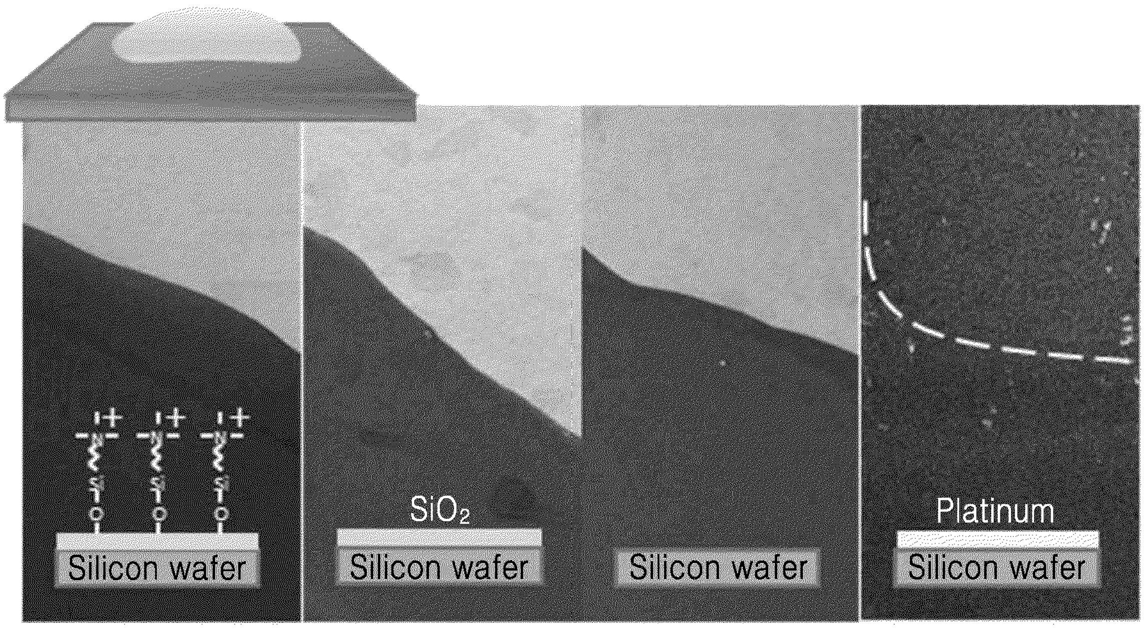
SiO2
Platinum
Silicon wafer
Silicon wafer
Silicon wafer
Silicon wafer

FIG. 14A

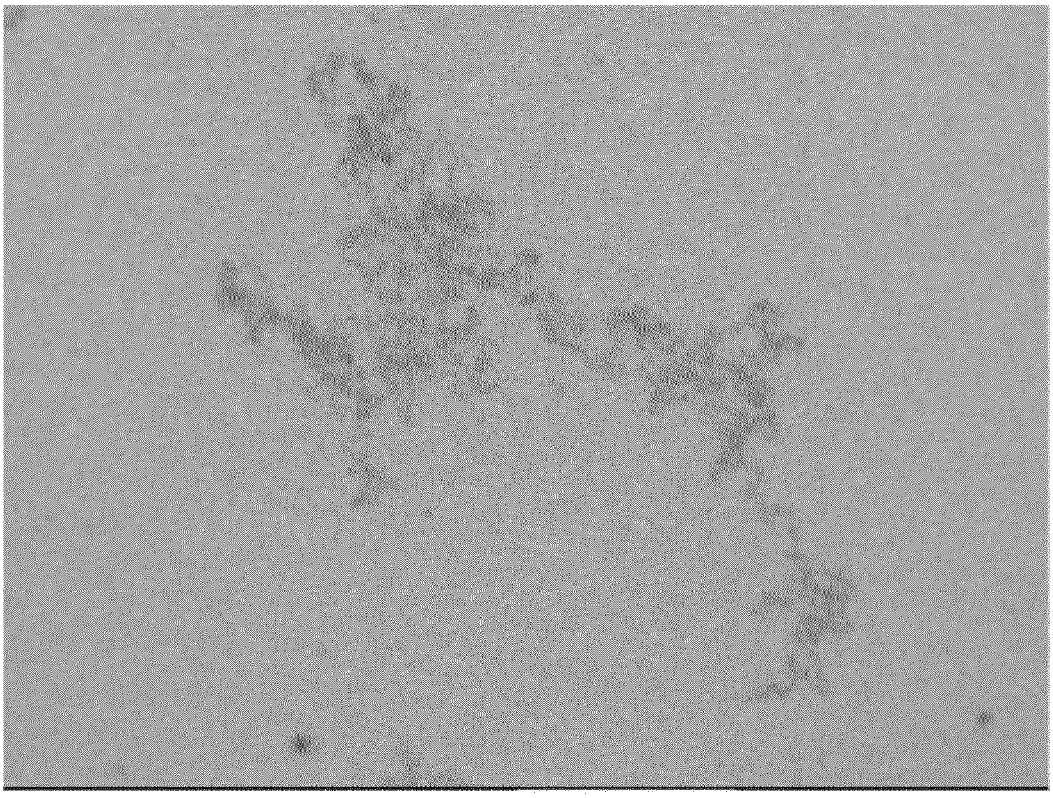

FIG. 14B

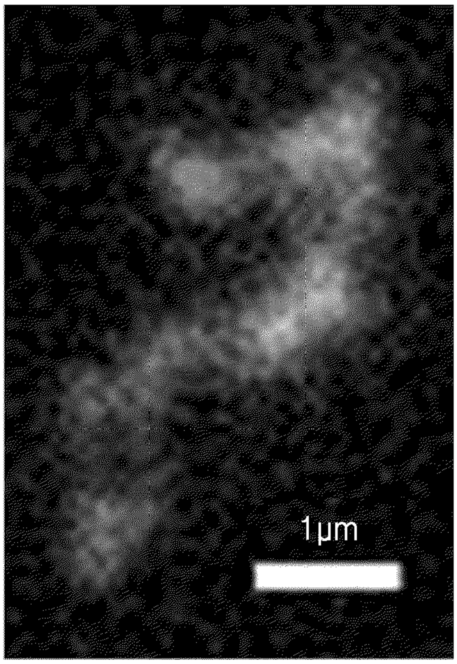

FIG. 15

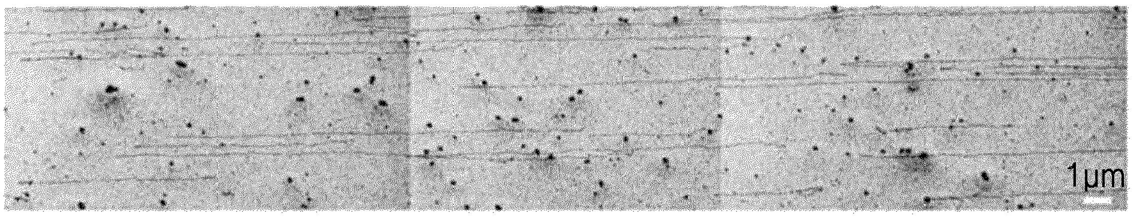


FIG. 16A

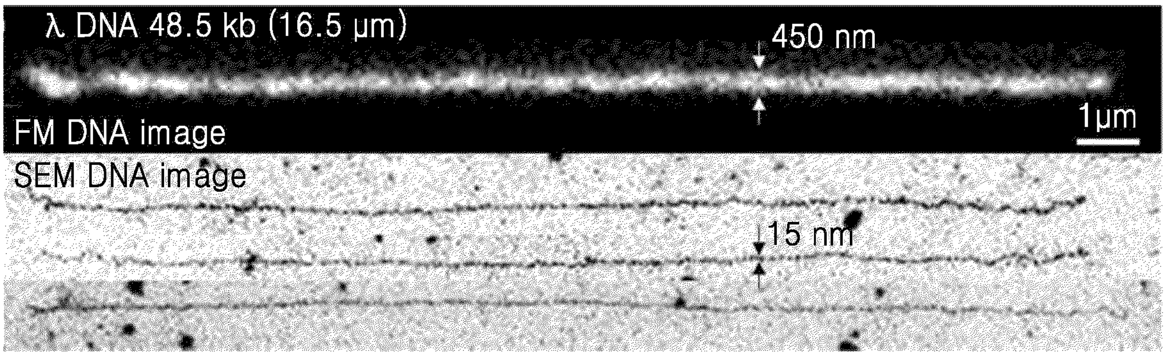

FIG. 16B

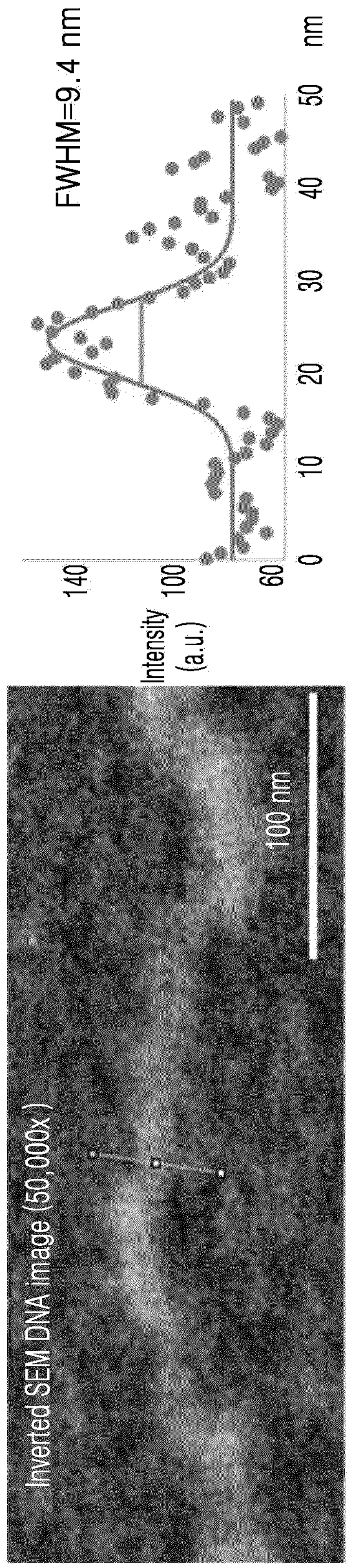
FWHM=9.4 nm
Intensity
(a.u.)
140
100
60
0
10
20
30
40
50
nm
Inverted SEM DNA image (50,000x)
100 nm

FIG. 16C

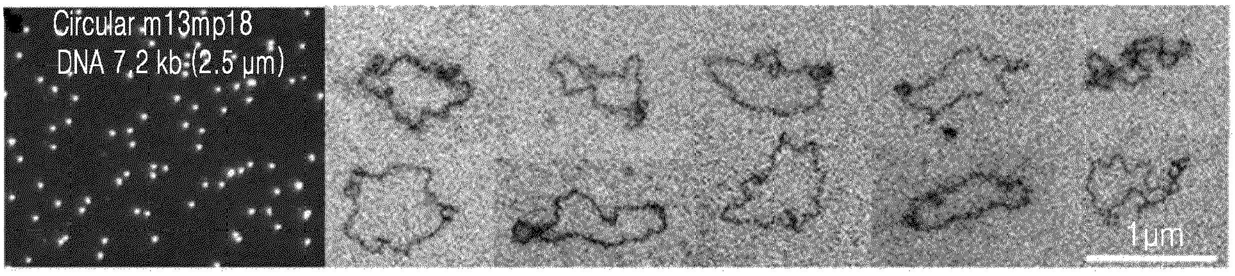


FIG. 17A

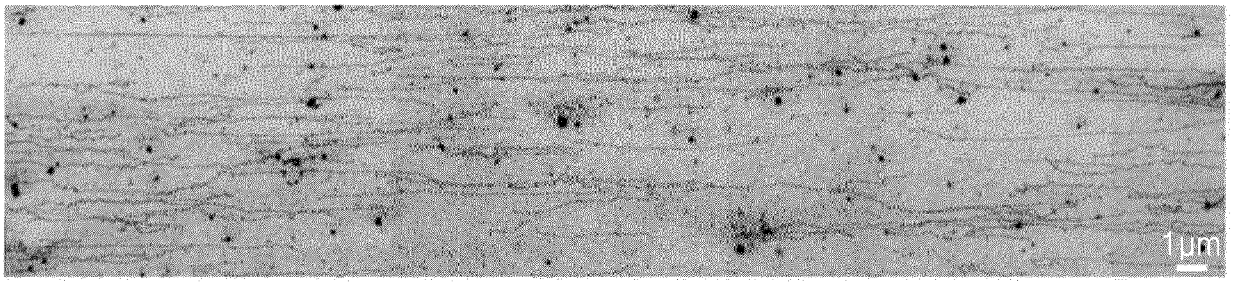


FIG. 17B

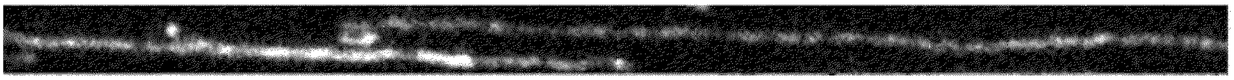

FIG. 17C

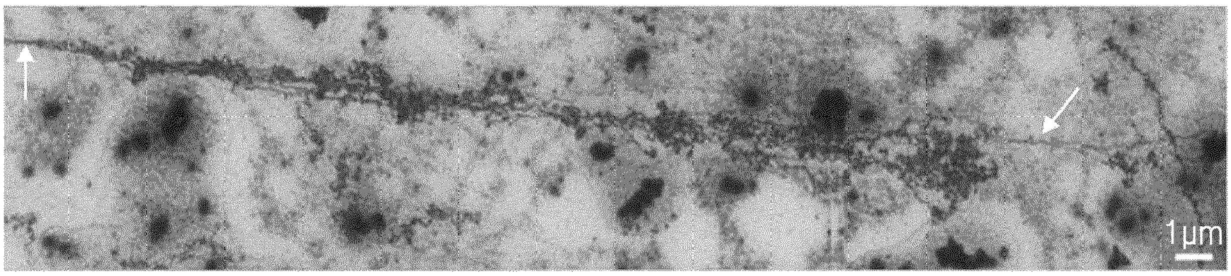

FIG. 18A

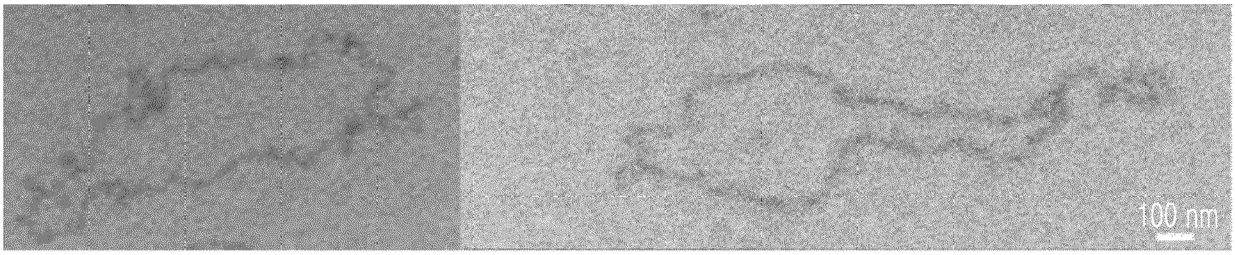


FIG. 18B

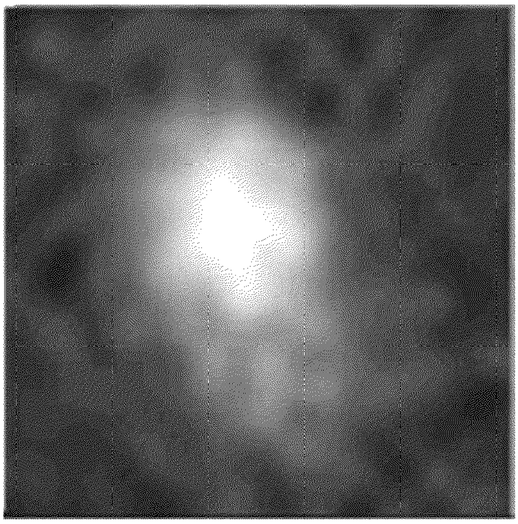

FIG. 19A

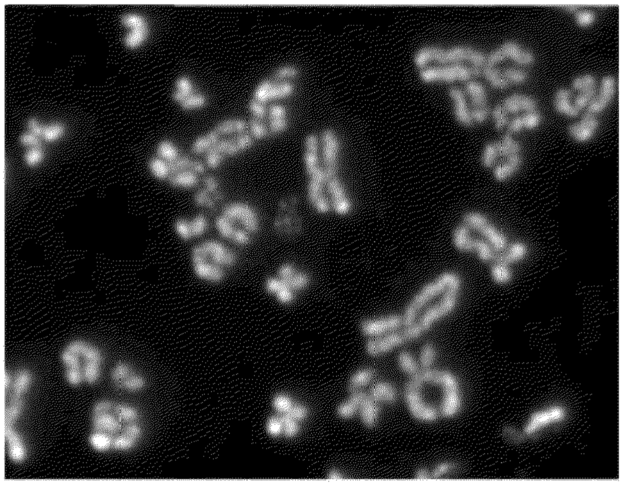

FIG. 19B

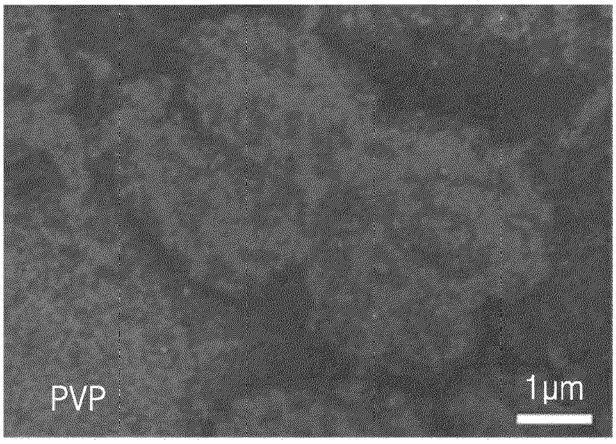


FIG. 19C

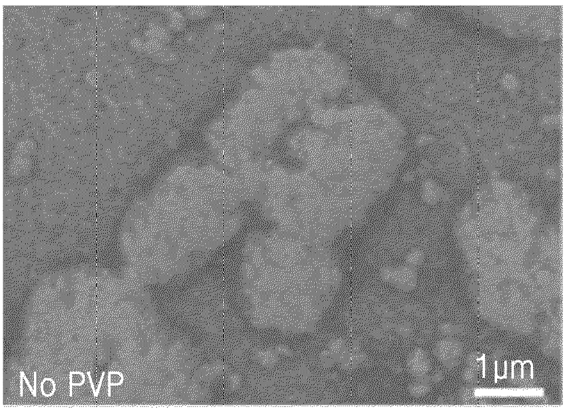

FIG. 20A

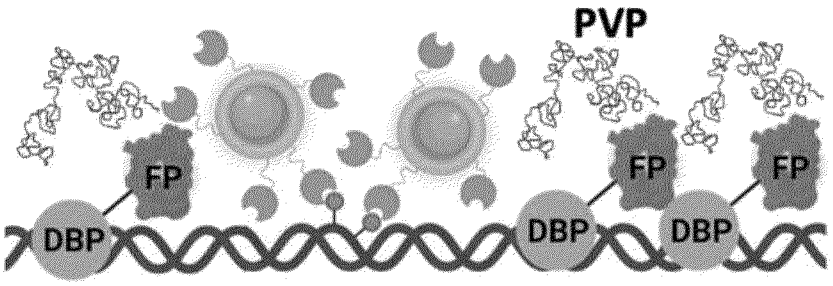


FIG. 20B

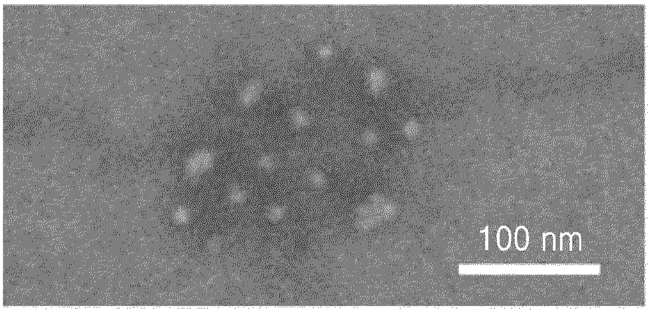


FIG. 20C

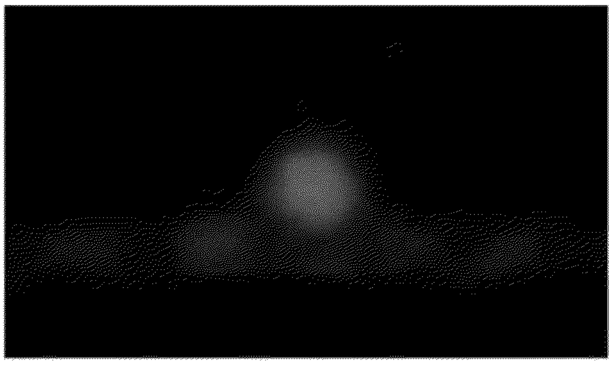

FIG. 21A

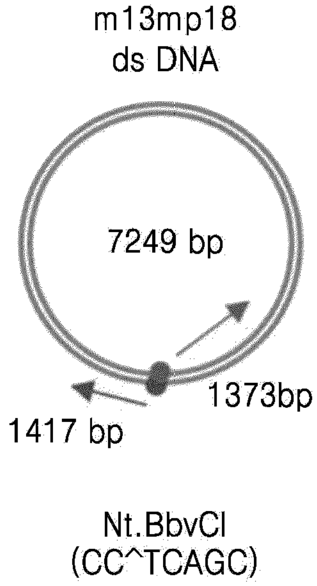


FIG. 21B

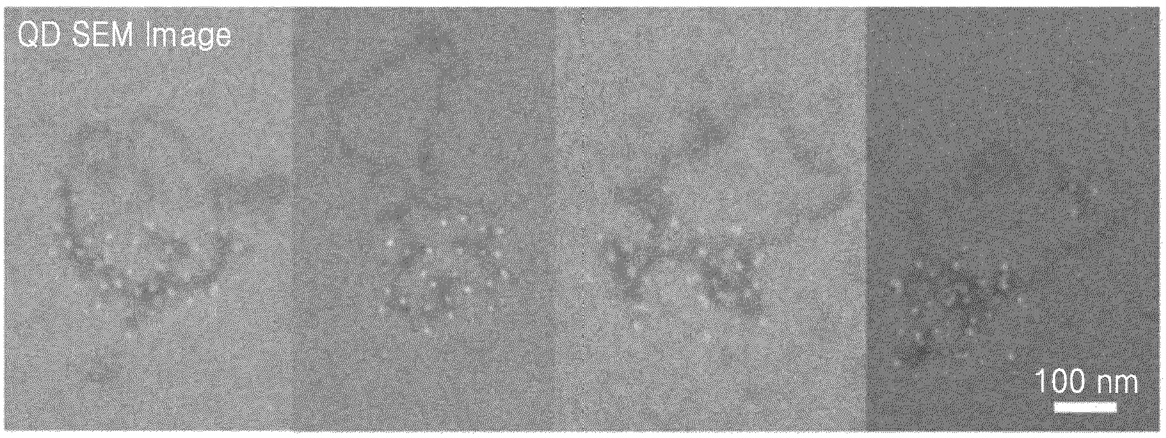

FIG. 21C

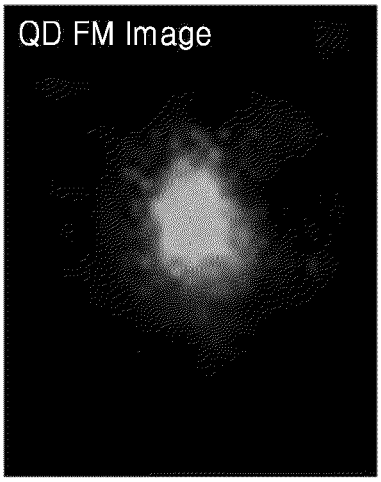

FIG. 22

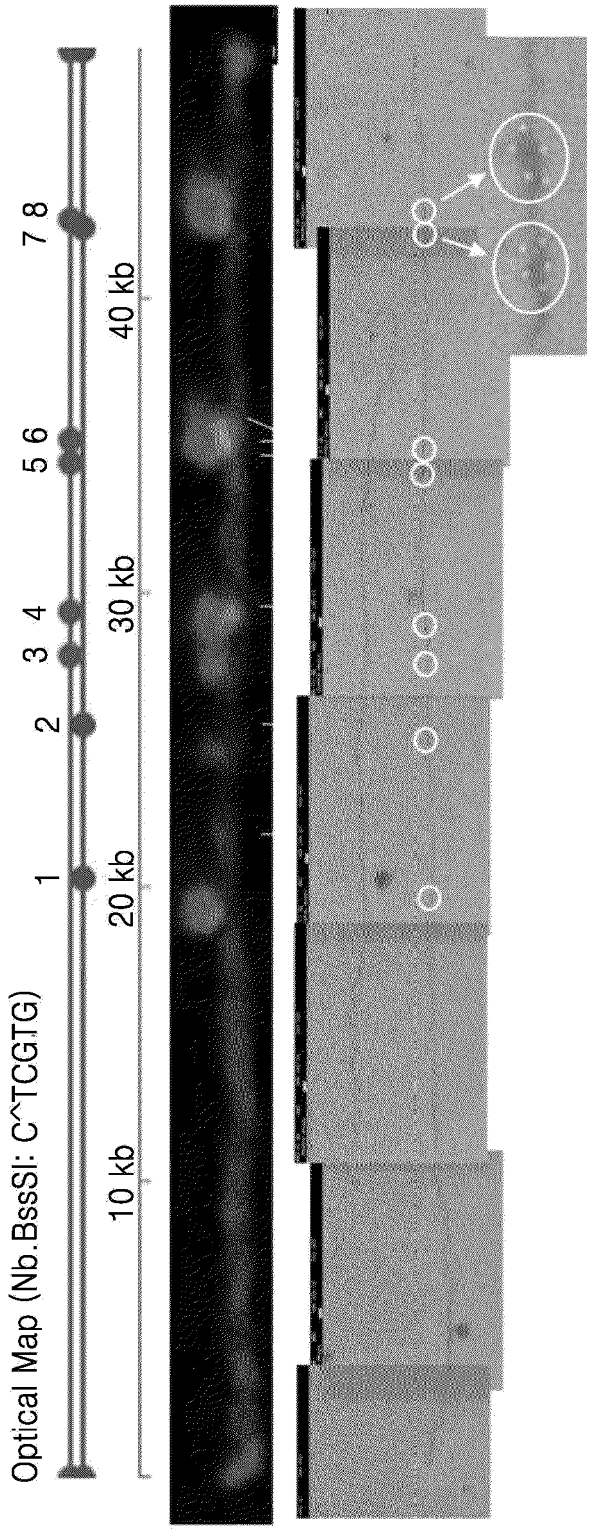

FIG. 23

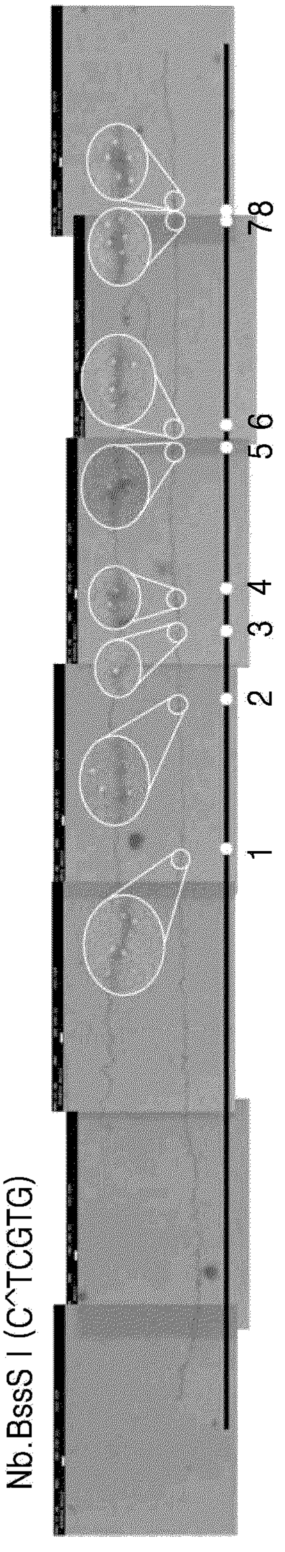

FIG. 24

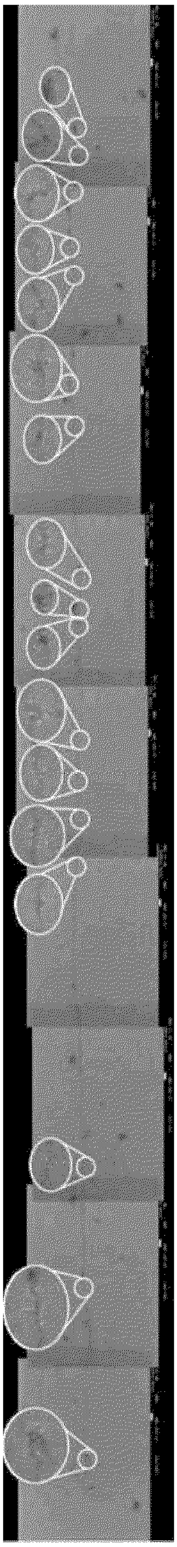

Europäisches Patentamt
European Patent Office
Office européen des brevets

# EUROPEAN SEARCH REPORT

Application Number
EP 25 16 3778

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 079 169 A (CHU STEVEN [US] ET AL) 7 January 1992 (1992-01-07) * the whole document * * col. 8, l. 22-33, l. 53-63; col. 11, l. 25-50 * | 1-15 | INV. C12Q1/6816 |
| X | WO 2012/056192 A1 (MIR KALIM [GB]; MARIE RODOLPHE [DK] ET AL.) 3 May 2012 (2012-05-03) * the whole document * * claims 16-18; p. 20, par. 1-3 from the bottom; p.2, par. 3; p.13, par. 2 from the bottom * | 1-15 | |
| X | WO 2009/052214 A2 (COMPLETE GENOMICS INC [US]; DRMANAC RADOJE [US]; DRMANAC SNEZANA [US]) 23 April 2009 (2009-04-23) * the whole document * * claims, para. 142, 185-186, 33, 34, 52, 172-174, 47, 95- 103, 105, 109-113 * | 1-15 | |
| Y | CHANYOUNG NOH.: "SEM image of DNA stained by protein and polymer without metals", CELL, [Online] 11 February 2024 (2024-02-11), XP093298504, Retrieved from the Internet: URL:https://www.cell.com/biophysj/pdf/S0006-3495(23)01626-0.pdf> [retrieved on 2025-07-24] * see "SEM image of DNA stained by protein and polymer without metals" * | 1-15 | TECHNICAL FIELDS SEARCHED (IPC) C12Q |

-/--

The present search report has been drawn up for all claims

1

| Place of search | Date of completion of the search | Examiner |
|---|---|---|
| Munich | 25 July 2025 | Celler, Jakub |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 25 16 3778

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | KYUNG-IL KIM ET AL: "DNA Binding Peptide Directed Synthesis of Continuous DNA Nanowires for Analysis of Large DNA Molecules by Scanning Electron Microscope", SMALL, vol. 13, no. 2, 3 November 2016 (2016-11-03), pages 1601926-1, XP055605516, Hoboken, USA ISSN: 1613-6810, DOI: 10.1002/smll.201601926 * the whole document * * p.2, right column; Fig. 1-3 * ----- | 1-15 | |
| Y,P | NOH CHANYOUNG ET AL: "Scanning Electron Microscopy Imaging of Large DNA Molecules Using a Metal-Free Electro-Stain Composed of DNA-Binding Proteins and Synthetic Polymers", ADVANCED SCIENCE, vol. 11, no. 28, 11 May 2024 (2024-05-11), pages e2309702-n/a, XP093298121, Germany ISSN: 2198-3844, DOI: 10.1002/advs.202309702 Retrieved from the Internet: URL:https://pmc.ncbi.nlm.nih.gov/articles/PMC11267313/pdf/ADVS-11-2309702.pdf> * the whole document * * Abstract, Fig. 1-4 * ----- -/-- | 1-15 | TECHNICAL FIELDS SEARCHED (IPC) |

The present search report has been drawn up for all claims

1

| Place of search | Date of completion of the search | Examiner |
|---|---|---|
| Munich | 25 July 2025 | Celler, Jakub |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 3

Europäisches Patentamt
European Patent Office
Office européen des brevets

# EUROPEAN SEARCH REPORT

Application Number
EP 25 16 3778

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | PRIYANNTH RAMASAMI SUNDHARBAABU ET AL: "Artificial Intelligence-Enhanced Analysis of Genomic DNA Visualized with Nanoparticle-Tagged Peptides under Electron Microscopy", SMALL, WILEY, HOBOKEN, USA, vol. 21, no. 12, 9 October 2024 (2024-10-09), page n/a, XP072854751, ISSN: 1613-6810, DOI: 10.1002/SMLL.202405065 * the whole document * ----- | | |

TECHNICAL FIELDS SEARCHED (IPC)

The present search report has been drawn up for all claims

1

| Place of search | Date of completion of the search | Examiner |
|---|---|---|
| Munich | 25 July 2025 | Celler, Jakub |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 3 of 3

# ANNEX TO THE EUROPEAN SEARCH REPORT ON EUROPEAN PATENT APPLICATION NO. EP 25 16 3778

This annex lists the patent family members relating to the patent documents cited in the above-mentioned European search report. The members are as contained in the European Patent Office EDP file on The European Patent Office is in no way liable for these particulars which are merely given for the purpose of information.

25-07-2025

| Patent document cited in search report | Publication date | Patent family member(s) | Publication date |
|---|---|---|---|
| US 5079169 A | 07-01-1992 | NONE | |
| WO 2012056192 A1 | 03-05-2012 | DK 2632592 T3 | 20-07-2015 |
| | | EP 2444157 A1 | 25-04-2012 |
| | | EP 2632592 A1 | 04-09-2013 |
| | | US 2013224736 A1 | 29-08-2013 |
| | | WO 2012055415 A1 | 03-05-2012 |
| | | WO 2012056192 A1 | 03-05-2012 |
| WO 2009052214 A2 | 23-04-2009 | US 2009111115 A1 | 30-04-2009 |
| | | WO 2009052214 A2 | 23-04-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020240036772


### Non-patent literature cited in the description


- 21 Fluorescent Protein-Based DNA Staining Dyes. *Molecules*, 2022, vol. 27 (16)
- Truncated TALE-FP as DNA Staining Dye in a High-salt Buffer. *Sci Rep-Uk*, 2019, vol. 9, 17197
- *Analyst*, 2019, vol. 144, 921-927
- AT-specific DNA visualization revisits the directionality of bacteriophage lambda DNA ejection. *Nucleic Acids Research*, 2023
- Negative nanopore sequencing for mapping biochemical processes on DNA molecules. *Chemical Communications*, 2023, vol. 59, 9388-9391
- Counting DNA molecules on a microchannel surface for quantitative analysis. *Talanta*, 2023, vol. 252, 123826
- **LEE et al.** *Nucleic Acids Research*, 2016, vol. 44 (1)
- Enhancement in Elastic Bending Rigidity of Polymer Loaded Reverse Microemulsion. *Langmuir*, 2017, vol. 33 (45), 13014-13026